# EUROPEAN PATENT APPLICATION

(11) **EP 4 588 942 A1**
(43) Date of publication of application: **23.07.2025**
(21) Application number: 23878678.4
(22) Date of filing: 26.06.2023
(51) Int. Cl.: C07K 19/00, C12N 15/62, C12N 15/86, C07K 16/10, G01N 33/569, G01N 33/68, C12Q 1/02, A61K 39/155

(54) **TRUNCATED RESPIRATORY SYNCYTIAL VIRUS F PROTEIN AND USE THEREOF**

(30) Priority: 17.10.2022 CN 202211267043
(71) Applicant: Xiamen University, Xiamen, Fujian 361005 (CN); Xiamen Innovax Biotech Co., Ltd., Xiamen, Fujian 361022 (CN)
(72) Inventor: ZHENG, Zizheng, Xiamen, Fujian 361005 (CN); LIN, Min, Xiamen, Fujian 361005 (CN); YIN, Yifan, Xiamen, Fujian 361005 (CN); WANG, Chen, Xiamen, Fujian 361005 (CN); ZHAO, Xiaomeng, Xiamen, Fujian 361005 (CN); CHEN, Li, Xiamen, Fujian 361005 (CN); ZHANG, Jun, Xiamen, Fujian 361005 (CN); XIA, Ningshao, Xiamen, Fujian 361005 (CN)
(74) Representative: Lavoix
(86) International application number: PCT/CN2023/102342
(87) International publication number: WO 2024/082681

(57) **Abstract**

Provided are a fusion protein, and a nucleic acid molecule comprising a nucleotide sequence encoding the fusion protein. The present invention also relates to a vaccine comprising the fusion protein or the nucleic acid molecule. Furthermore, the present invention also relates to a method for preventing and/or treating RSV infections or diseases and/or symptoms caused by RSV infections by means of using the fusion protein, nucleic acid molecule and vaccine.

## Description

### Technical Field

The present application relates to the field of biomedicine, and specifically, the present application relates to a fusion protein and a nucleic acid molecule comprising a nucleotide sequence encoding the fusion protein. The present application also relates to a vaccine comprising the fusion protein or the nucleic acid molecule. Further, the present invention also relates to a method of using the fusion protein, the nucleic acid molecule and the vaccine for preventing and/or treating a RSV infection or disease and/or a symptom caused by RSV infection.

### Background Art

Respiratory syncytial virus (RSV) is one of the most important pathogens causing lower respiratory tract infections in infants and young children worldwide. According to statistics, 33 million children under the age of 5 are infected with RSV each year in the world, causing nearly 160,000 deaths. After RSV infection, continuous immunity may not be obtained, and repeated infections may occur. More than 99% of children under the age of 2 have been infected with RSV at least once, and 70% of patients have hospitalized infections complicated by bronchiolitis, pneumonia and asthma. In addition to infants and children, elderly people with low immunity and immunosuppression are also at high risk of RSV infection. The elderly often develop obstructive pulmonary disease with cardiopulmonary complications. Despite the serious disease and economic burden on the world, there is still no safe and effective vaccine for RSV.

RSV belongs to the genus *Pneumovirus* and the family *Pneumoviridae,* and is an enveloped single-stranded negative-strand RNA virus. The genome is about 15.2kb long, transcribes ten genes, and encodes a total of eleven proteins. Among them, the adhesion glycoprotein G and membrane fusion protein F are located on the surface of viral membrane. Compared with the G protein, the F protein has an advantage in conservatism, so it is considered to be the most important protective antigen of RSV.

The F protein of RSV belongs to the type I integral membrane protein, and its precursor protein F0 consists of 574 amino acids, and three F0s form a trimer through hydrophobic interaction. When passing through the Golgi apparatus, it is cleaved by the host's furin protease between amino acids 109 and 110 and between amino acids 136 and 137. After cleavage, the F protein releases a short peptide P27 containing 27 amino acids. The remaining two segments F2 and F1 are linked by two disulfide bonds to form a mature F protein, which is displayed in budding form on the surface of cell membrane or virion. At this time, the F protein is in a high-energy metastable state and is very unstable, called pre-F. At the N-terminal of F1, there is a highly hydrophobic fusion peptide FP, which is located in the hydrophobic cavity of the trimer. Under the triggering of currently unknown factors, the N-terminal of F1 undergoes a series of drastic structural changes, and this process allows FP to be inserted into the cell membrane surface to help the virus complete membrane fusion and infect cells, and also causes the pre-F structure to become a stable low-energy post-F conformation.

In 2013, McLellan et al. first obtained a stable pre-F protein through point mutation and named it DS-Cav1. Subsequently, several pre-F proteins were reported, including DS-Cav1-Cys-zipper, SC-DM and SC-TM. Compared with post-F protein, it has been proven that these pre-F proteins are capable of activating significantly higher level of neutralization antibody titer. RSV pre-F protein can be divided into two parts: a membrane-proximal "handle" region, including domain I and domain II, which is mainly composed of β-strand structure and terminates at the C-terminal helix that enters into the membrane; and a membrane-distal part, i.e., RSV F "head" region, including domain III, which is mainly α-helix. The RSV F head contains at least two epitopes associated with neutralization antibody. Site Ø and site V are located at the top of the pre-F trimer and can be recognized by strong neutralization antibodies including D25, AM22, 5C4 and hRSV90, respectively. Site II is located in the middle of the pre-F trimer and is the binding site of the commercial antibody palivizumab. Site I and site IV, which are also recognized by low-efficiency antibodies, are located in the handle region of the pre-F protein. The handle region of the pre-F protein accounts for 55% of the total surface area of the trimer, which may compete with the high-neutralization epitopes site Ø or site V in the head region, resulting in a weakened antibody response to the high-neutralization epitopes.

The main purpose of the research on RSV vaccine with F protein as the main protective antigen is to produce an antibody with high neutralization titer, and the improvement of the immunogenicity of RSV vaccine with F protein as the protective antigen is of great significance to the development of RSV vaccine.

### Contents of the present invention

### Fusion protein containing truncated moieties

In the first aspect, the present application provides a fusion protein, which comprises a first truncate, a second truncate, and a linker linking the first truncate and the second truncate; wherein,
as compared to the F2 protein of wild respiratory syncytial virus (RSV), the first truncate has 5 to 25 amino acids (e.g., 5 to 8, 9 to 12, 13 to 16, 17 to 20, 21 to 25) truncated at the N-terminal, and 3 to 5 amino acids (e.g., 3, 4, 5) truncated at the C-terminal of the F2 protein of wild RSV;
as compared to the F1 protein of wild RSV, the second truncate has 7 to 9 amino acids (e.g., 7, 8, 9) truncated at the N-terminal, and 238 to 268 amino acids (e.g., 238 to 241, 242 to 245, 246 to 249, 250 to 253, 254 to 257, 258 to 261, 262 to 265, 266 to 268) truncated at the C-terminal of the F1 protein of wild RSV.

In certain embodiments, the truncation length of the C-terminal of the second truncate has a certain range (i.e., truncation of 238 to 268 amino acids), and the truncation length of the N-terminal of the first truncate has a certain range (i.e., truncation of 5 to 25 amino acids), because within these ranges, the first truncate and the second truncate can still keep intact several protein secondary structures (such as β2/β7, α1 and α4, etc.) that maintain conformational stability.

In some embodiments, compared to the F2 protein of wild RSV, the first truncate has 5 or 25 amino acids truncated at the N-terminal and 4 amino acids truncated at the C-terminal of the F2 protein of wild RSV.

In some embodiments, the F2 protein of wild RSV has an amino acid sequence as set forth in SEQ ID NO: 13.

In some embodiments, the first truncate has an amino acid sequence as set forth in SEQ ID NO: 16 or 17.

In some embodiments, compared to the F1 protein of wild RSV, the second truncate has 8 or 9 amino acids truncated at the N-terminal and 252 or 268 amino acids truncated at the C-terminal of the F1 protein of wild RSV.

In some embodiments, the F1 protein of wild RSV has an amino acid sequence as set forth in SEQ ID NO: 12.

In some embodiments, the second truncate has an amino acid sequence as set forth in SEQ ID NO: 14 or 15.

In some embodiments, the linker comprises at least 1 (e.g., 1, 2) proline.

In some embodiments, the linker has 3 to 20 amino acids (e.g., 3, 4, 5, 6, 7, 8, 9, 10, 15, 20). In some embodiments, the linker has 5 to 8 amino acids.

In some embodiments, the linker comprises a plurality of (e.g., 2, 3, 4, 5, 6, 7) glycine and at least 1 (e.g., 1, 2) proline.

In some embodiments, the linker has a sequence as set forth in SEQ ID NO: 2.

In some embodiments, the first truncate is located at the N-terminal of the linker.

In some embodiments, the second truncate is located at the C-terminal of the linker.

In some embodiments, the fusion protein comprises, from the N-terminal to the C-terminal, the first truncate, the linker, the second truncate.

In some embodiments, the fusion protein has an amino acid sequence as set forth in SEQ ID NO: 3 or 4.

### Fusion protein containing truncated mutants

In certain embodiments, the fusion protein further comprises a substitution, deletion or addition of one or more amino acids (e.g., a substitution, deletion or addition of 1, 2, 3, 4, 5, 6, 7, 8, 9 or 10 amino acids).

In certain embodiments, the first truncate and/or the second truncate of the fusion protein comprises a substitution, deletion or addition of one or more amino acids (e.g., a substitution, deletion or addition of 1, 2, 3, 4, 5, 6, 7, 8, 9 or 10 amino acids).

In certain embodiments, the sequence of the fusion protein has a substitution, deletion or addition of one or more amino acids (e.g., a substitution, deletion or addition of 1, 2, 3, 4, 5, 6, 7, 8, 9 or 10 amino acids) as compared to the sequence as set forth in any one of SEQ ID NOs: 3 to 7;
In certain embodiments, the substitution is a conservative substitution.

In some embodiments, the sequence of the fusion protein has a substitution, deletion or addition of one or more amino acids (e.g., a substitution, deletion or addition of 1, 2, 3, 4, 5, 6, 7, 8, 9 or 10 amino acids) as compared to the sequence as set forth in SEQ ID NO: 4.

In some embodiments, compared to the sequence as set forth in SEQ ID NO: 4, the sequence of the fusion protein has a substitution of amino acid at the position corresponding to the position 29 of SEQ ID NO: 4, and, the sequence of the fusion protein also has a substitution of amino acid at any one of the positions corresponding to the positions 47 to 214 (e.g., positions 47, 212, 214, 88, 94, 114, 101) of SEQ ID NO: 4.

In certain embodiments, as compared to the sequence as set forth in SEQ ID NO: 4, the sequence of the fusion protein has a substitution of amino acid at the position corresponding to the position 114 of SEQ ID NO: 4, and the sequence of the fusion protein also has a substitution of amino acid at any one of the positions corresponding to the positions 29 to 94 of SEQ ID NO: 4.

In certain embodiments, as compared to the sequence as set forth in SEQ ID NO: 4, the sequence of the fusion protein has a substitution of one or more amino acids at the following positions corresponding to the position 29, position 47, position 212, position 214, position 88, position 94, position 114 and position 101 of SEQ ID NO: 4.

In certain embodiments, as compared to the sequence as set forth in SEQ ID NO: 4, the sequence of the fusion protein has a substitution of amino acid at the positions corresponding to the positions 29 and 212 of SEQ ID NO: 4.

In certain embodiments, as compared to the sequence as set forth in SEQ ID NO: 4, the sequence of the fusion protein has a substitution of amino acid at the positions corresponding to the positions 29 and 214 of SEQ ID NO: 4.

In certain embodiments, as compared to the sequence as set forth in SEQ ID NO: 4, the sequence of the fusion protein has a substitution of amino acid at the positions corresponding to the position 29 and position 114 of SEQ ID NO: 4.

In certain embodiments, as compared to the sequence as set forth in SEQ ID NO: 4, the sequence of the fusion protein has a substitution of amino acid at the positions corresponding to the position 29, position 47, position 101 and position 214 of SEQ ID NO: 4.

In certain embodiments, as compared to the sequence as set forth in SEQ ID NO: 4, the sequence of the fusion protein has a substitution of amino acid at the positions corresponding to the position 88, position 101, position 114 and position 214 of SEQ ID NO: 4.

In certain embodiments, as compared to the sequence as set forth in SEQ ID NO: 4, the sequence of the fusion protein has a substitution of amino acid at the positions corresponding to the positions 94 and position 114 of SEQ ID NO: 4.

In certain embodiments, the substitution at position 29 is to substitute isoleucine I with leucine L.

In certain embodiments, the substitution at position 212 is to substitute valine V with methionine M.

In certain embodiments, the substitution at position 214 is to substitute alanine A with leucine L.

In certain embodiments, the substitution at position 94 is to substitute valine V with isoleucine I.

In certain embodiments, the substitution at position 47 is to substitute methionine M with alanine A.

In certain embodiments, the substitution at position 114 is to substitute tyrosine Y with isoleucine I.

In certain embodiments, the substitution at position 101 is to substitute valine V with isoleucine I.

In certain embodiments, the substitution at position 214 is to substitute alanine A with leucine L.

In certain embodiments, the substitution at position 88 is to substitute amino acid leucine L with alanine A.

In certain embodiments, the fusion protein has an amino acid sequence as set forth in any one of SEQ ID NOs: 28 to 34.

In some embodiments, the fusion protein further comprises: a signal peptide, a multimerization motif (e.g., a trimerization motif) and/or a membrane anchoring region.

In some embodiments, the membrane anchoring region has an amino acid sequence as set forth in SEQ ID NO: 8.

In some embodiments, the signal peptide has an amino acid sequence as set forth in SEQ ID NO: 9.

In some embodiments, the multimerization motif has an amino acid sequence as set forth in SEQ ID NO: 21 or 22.

In some embodiments, the signal peptide is located at the N-terminal of the first truncate. In some embodiments, the signal peptide is linked to the first truncate via a first linker peptide.

In some embodiments, the membrane anchoring region is located at the C-terminal of the second truncate. In some embodiments, the membrane anchoring region is linked to the second truncate via a second linker peptide.

In some embodiments, the multimerization motif is located at the C-terminal of the second truncate. In some embodiments, the multimerization motif is linked to the second truncate via a second linker peptide.

In some embodiments, the first linker peptide and the second linker peptide each independently comprise one or more (e.g., 1, 2, or 3) sequences represented by (GₘS)ₙ, wherein m is an integer selected from 1 to 6, and n is an integer selected from 1 to 6. In some embodiments, m is 3, 4, or 5. In some embodiments, n is 1 or 2. In some embodiments, the first linker peptide and the second linker peptide have a sequence as set forth in SEQ ID NO: 18.

In some embodiments, the fusion protein comprises, from the N-terminal to the C-terminal, the signal peptide, the first linker peptide, the first truncate, the linker, the second truncate, the second linker peptide, the membrane anchoring region.

In certain embodiments, the fusion protein has a sequence as set forth in SEQ ID NO: 19, 20, 45 or 46.

On the other hand, the present application provides a nucleic acid molecule, comprising a nucleotide sequence encoding the fusion protein as described above.

In certain embodiments, the nucleotide sequence is codon-optimized or not codon-optimized according to the codon preference of a host cell.

On the other hand, the present application provides a vector, comprising the nucleic acid molecule as described above.

In certain embodiments, the vector is a viral vector.

In certain embodiments, the viral vector is selected from the group consisting of: influenza virus vector, enterovirus vector, retrovirus vector, adenovirus vector, adeno-associated virus vector, herpes virus vector, poxvirus vector, baculovirus vector, papillomavirus vector, or papovavirus vector.

On the other hand, the present application provides a host cell, comprising the fusion protein as described above or the nucleic acid molecule as described above or the vector as described above.

In certain embodiments, the host cell is selected from the group consisting of prokaryotic cell (e.g., *Escherichia coli* cell) and eukaryotic cell.

In some embodiments, the eukaryotic cell is a mammalian cell, such as a mouse cell or a human cell.

In some embodiments, the fusion protein comprises a membrane anchoring region and is displayed on the surface of the cell membrane of the host cell.

On the other hand, the present application provides a method for expressing or producing the fusion protein as described above, the method comprising culturing the host cell as described above under a condition that allow protein expression, and optionally, recovering or purifying the expressed fusion protein.

On the other hand, the present application provides a kit, comprising an antigen component and a carrier component capable of displaying the antigen component; wherein,
the antigen component comprises (i) the fusion protein as described above, (ii) the nucleic acid molecule as described above, and/or (iii) an mRNA product transcribed from the nucleic acid molecule as described above;
the carrier component is selected from the group consisting of: nanomaterial (e.g., lipid nanoparticle, protein nanoparticle, polymer nanoparticle, inorganic nanocarrier and biomimetic nanoparticle), bacterial outer-membrane vesicles (OMVs), multimerization motif, virus-like particle (VLP), or any combination thereof.

In some embodiments, the antigen component and the carrier component in the kit are provided separately or in a complex formed thereof.

In certain embodiments, the antigen component is a monomer or a multimer (e.g., a dimer, a trimer, a tetramer, a pentamer).

In certain embodiments, the multimerization motif and/or VLP in the kit is provided in the form of a protein or a nucleic acid molecule comprising a nucleotide sequence encoding the protein.

In certain embodiments, the multimerization motif has an amino acid sequence as set forth in SEQ ID NO: 21 or 22.

In certain embodiments, the VLP is assembled by proteins obtained from RSV, hepatitis E virus (HEV), hepatitis B virus (HBV), human papillomavirus (HPV), or human immunodeficiency virus (HIV).

On the other hand, the present application provides a pharmaceutical composition, comprising a pharmaceutically acceptable carrier and/or excipient, and any one or more selected from the following items (1) to (4):
(1) the fusion protein as described above;
(2) the nucleic acid molecule as described above;
(3) the vector as described above;
(4) the host cell as described above.

In another aspect, the present application provides a method for producing a vaccine against respiratory syncytial virus (RSV), which method comprises providing the kit or a pharmaceutical composition as described above, and is formulated into a pharmaceutically acceptable vaccine.

In another aspect, the present application provides a vaccine, comprising the fusion protein as described above, or the nucleic acid molecule as described above, or an mRNA product transcribed from the nucleic acid molecule as described above, or the vector as described above.

In certain embodiments, the vaccine is prepared from the kit or pharmaceutical composition as described above.

In certain embodiments, the vaccine further comprises an adjuvant.

In certain embodiments, the vaccine as described above can be administered to a subject, such as a human subject. The total dose of the fusion protein in the vaccine for single administration can be, for example, from about 0.01 µg to about 10 mg, such as 1 µg to 1 mg, such as 10 µg to 100 µg. Determining the dose to be administered can be determined experimentally, and determining the dose is routine for those skilled in the art.

On the other hand, the present application provides a method for inducing an antibody against RSV, the method comprising performing an administration (e.g., injection) of an effective amount of the fusion protein as described above, or the nucleic acid molecule as described above, or the vector as described above, or the host cell as described above, or the pharmaceutical composition as described above, or the vaccine as described above to a cell in vitro or to a subject in vivo.

In certain embodiments, the antibody is a neutralization antibody.

In certain embodiments, the administration comprises parenteral administration, such as intradermal, intramuscular, subcutaneous, transdermal administration, or mucosal administration, such as intranasal, oral administration, or the like. In certain embodiments, the composition is administered by intramuscular injection.

In addition, the fusion protein of the present invention can be used as a diagnostic tool, for example, to detect the immune status of a subject by determining whether there is an antibody capable of binding to the fusion protein in the serum of the subject.

Therefore, on the other hand, the present application provides a method for detecting whether a subject is infected with RSV in vitro, the method comprising: contacting a biological sample obtained from the subject with the fusion protein as described above; and detecting the presence of a complex formed by the fusion protein and an antibody from the biological sample.

In certain embodiments, the subject is a mammal, for example, a mouse, a human.

In certain embodiments, the biological sample is selected from the group consisting of whole blood, serum, plasma, or any combination thereof.

On the other hand, the present application provides a method for screening a candidate medicament capable of inhibiting RSV infection in cells, the method comprising: contacting a host cell with the candidate medicament before, simultaneously or after contacting the fusion protein as described above, or the vector as described above, or the pharmaceutical composition as described above, or the vaccine as described above with the host cell.

On the other hand, the present application provides the use of the fusion protein as described above, or the nucleic acid molecule as described above, or the vector as described above, or the host cell as described above, or the pharmaceutical composition as described above, or the vaccine as described above in the manufacture of a kit for inducing an immune response to RSV in a subject.

In certain embodiments, the immune response comprises inducing the subject to produce an antibody against RSV.

In certain embodiments, the antibody is a neutralization antibody.

In certain embodiments, the subject is a mammal, for example, a mouse, a human.

On the other hand, the present application provides the use of the fusion protein as described above, or the nucleic acid molecule as described above, or the vector as described above, or the host cell as described above, or the pharmaceutical composition as described above, or the vaccine as described above in the manufacture of a kit for preventing and/or treating a RSV infection or a disease and/or symptom caused by RSV infection.

In certain embodiments, the subject is a mammal, for example, a mouse, a human. In certain embodiments, the disease and symptom caused by RSV infection are selected from bronchiolitis, pneumonia, asthma, obstructive pulmonary disease, and cardiopulmonary complication.

### Definition of terms

As used herein, the terms "respiratory syncytial virus" and "RSV" have the same meaning, which is a virus belonging to the family *Pneumoviridae* and the genus *Pneumovirus.* The RSV genome is about 15Kb in length, contains 10 genes, and encodes 11 proteins, including 8 structural proteins (F, G, M2-1, M2-2, SH, N, P, L) and 3 non-structural proteins (NS1, NS2, NS3). Among them, fusion protein (F) and attachment protein (G) are two main envelope glycoproteins. F protein is a type I glycoprotein. After being cleaved by cell proteases into F1 and F2, it has biological activity and can fuse the viral envelope with the host cell membrane to form a multinucleated giant cell. The sequence of F protein can be obtained from public databases (e.g., GenBank database). In certain embodiments, the amino acid sequence of wild F protein is set forth in SEQ ID NO: 1.

As used herein, the terms "wild" or "natural" are used interchangeably. When these terms are used to describe nucleic acid molecules, polypeptides or proteins, they indicate that the nucleic acid molecules, polypeptides or proteins exist in nature, are found in nature, and have not been artificially modified or processed. As used herein, the F1/F2 proteins of wild respiratory syncytial virus (RSV) refer to naturally occurring, biologically active F1/F2 proteins. Those skilled in the art can easily obtain the amino acid sequences of F1 and F2 proteins from various public databases (e.g., GenBank database). For example, the amino acid sequence of the F2 protein of wild RSV can be set forth in SEQ ID NO: 13. The amino acid sequence of the F1 protein of wild RSV can be set forth in SEQ ID NO: 12.

As used herein, the term "X amino acids truncated at the C-terminal" means that the most distal consecutive X amino acids at the C-terminal are truncated. Similarly, the term "X amino acids truncated at the N-terminal" means that the most distal consecutive X amino acids at the N-terminal are truncated.

As used herein, the term "vector" refers to a nucleic acid carrier into which a polynucleotide can be inserted. When a vector can express a protein encoded by an inserted polynucleotide, the vector is called an expression vector. The vector can be introduced into a host cell by transformation, transduction or transfection so that the genetic material elements it carries are expressed in the host cell. Vectors are well known to those skilled in the art, including but not limited to: plasmid; phagemid; cosmid; artificial chromosome, such as yeast artificial chromosome (YAC), bacterial artificial chromosome (BAC) or P1-derived artificial chromosome (PAC); bacteriophage, such as λ phage or M13 phage, and animal virus, etc. Animal viruses that can be used as vectors include but are not limited to retrovirus (including lentivirus), adenovirus, adeno-associated virus, herpes virus (e.g., herpes simplex virus), poxvirus, baculovirus, papillomavirus, papovavirus (e.g., SV40). A vector can contain a variety of elements that control expression, including but not limited to promoter sequence, transcription start sequence, enhancer sequence, selection element and reporter gene. In addition, the vector may also contain a replication origin site.

As used herein, the term "host cell" refers to a cell that can be used to introduce a vector, including but not limited to prokaryotic cell such as *Escherichia coli* or *Bacillus subtilis,* fungal cell such as yeast cell or *Aspergillus,* insect cell such as *S2 Drosophila* cell or Sf9, or animal cell such as fibroblast, CHO cell, COS cell, NSO cell, HeLa cell, BHK cell, HEK 293 cell or human cell.

As known to those skilled in the art, codons have degeneracy. That is, during the translation of a protein, each amino acid can correspond to one or more codons, for example, up to 6 codons. Different species have great differences in the use of degenerate codons encoding a certain amino acid, and have different preferences. This preference phenomenon is called "codon preference". Therefore, as used herein, the term "codon preference" refers to the situation where a species prefers to use certain specific codons to encode amino acids. Optimizing the sequence of a nucleic acid molecule according to codon preference is particularly advantageous in some cases, for example, it may help to increase the expression level of the protein encoded by the nucleic acid molecule. For example, when using *E. coli* (or human cell) to express a protein or fragment thereof, it would be potentially advantageous to optimize the nucleic acid sequence encoding the protein or fragment thereof for the codon preference of *E. coli* (or human cell).

As used herein, the term "virus-like particle (VLP)" is a polymer particle which structure is similar or dissimilar to a natural virus particle. In some embodiments, VLP is a natural virus particle. In some embodiments, VLP is a virus-like particle assembled from proteins. It has been shown that proteins (e.g., capsid proteins, surface proteins, envelope proteins) of some viruses (e.g., RSV, HBV, HEV, HPV) can spontaneously form VLPs after recombinant expression in an appropriate expression system.

As used herein, the term "pharmaceutically acceptable" means that it is recognized in the pharmaceutical field that it can be used in animals, especially in humans. As used herein, the term "pharmaceutically acceptable carrier and/or excipient" refers to a carrier and/or excipient that is pharmacologically and/or physiologically compatible with a subject and an active ingredient, which is well known in the art (see, for example, Remington's Pharmaceutical Sciences. Edited by Gennaro AR, 19th ed. Pennsylvania: Mack Publishing Company, 1995), and includes, but is not limited to: pH adjuster (including, but not limited to, phosphate buffer), surfactant (including, but not limited to, cationic, anionic or nonionic surfactant, such as Tween-80), adjuvant, ionic strength enhancer (including, but not limited to, sodium chloride), diluent, excipient, medium for containing or administering therapeutic agent, and any combination thereof.

As used herein, the pharmaceutically acceptable carrier can be a sterile liquid, such as water and oil, including oil derived from petroleum, animal or plant, or synthetic oil, such as peanut oil, soybean oil, mineral oil, sesame oil, and the like. When the pharmaceutical composition is administered intravenously, a physiological saline is the preferred carrier. Saline solutions and aqueous dextrose and glycerol solutions can also be used as liquid carriers, particularly for injectable solutions.

As used herein, pharmaceutically acceptable excipient may include starch, glucose, lactose, sucrose, gelatin, malt, rice, flour, chalk, silica gel, sodium stearate, glycerol monostearate, talc, sodium chloride, milk powder, glycerol, propylene, ethylene glycol, water, ethanol, etc. If necessary, the pharmaceutical composition may also contain a wetting agent, or an emulsifier such as sodium hyaluronate, or a pH buffer. The pharmaceutical composition may take the form of a solution, a suspension, an emulsion, a tablet, a pill, a capsule, a powder, a sustained-release formulation, etc.

As used herein, the term "subject" refers to a mammal, including but not limited to, human, rodent (mouse, rat, guinea pig), dog, horse, cow, cat, pig, monkey, chimpanzee, etc. Preferably, the subject is a human.

As used herein, the term "effective amount" refers to an amount sufficient to obtain or at least partially obtain the desired effect. For example, a disease prevention effective amount refers to an amount sufficient to prevent, arrest, or delay the occurrence of a disease; a disease treatment effective amount refers to an amount sufficient to cure or at least partially prevent a disease and complication thereof in a patient already suffering from the disease. Determination of such an effective amount is within the skill of technicians in the art. The amount effective for therapeutic use will depend, for example, on the severity of the disease to be treated, the overall state of the patient's own immune system, the patient's general condition such as age, weight and sex, the mode of administration of drug, and other treatments administered simultaneously, etc.

As used herein, the term "conservative substitution" refers to an amino acid substitution that does not adversely affect or change the expected properties of the protein/polypeptide comprising the amino acid sequence. For example, conservative substitutions can be introduced by standard techniques known in the art such as site-directed mutagenesis and PCR-mediated mutagenesis. Conservative substitutions of amino acids include substitutions of amino acid residues with amino acid residues having similar side chains, such as substitutions with residues that are physically or functionally similar to the corresponding amino acid residues (e.g., having similar size, shape, charge, chemical properties, including the ability to form covalent bonds or hydrogen bonds, etc.). Families of amino acid residues with similar side chains have been defined in the art. These families include amino acids with basic side chains (e.g., lysine, arginine, and histidine), acidic side chains (e.g., aspartic acid, glutamic acid), uncharged polar side chains (e.g., glycine, asparagine, glutamine, serine, threonine, tyrosine, cysteine, tryptophan), non-polar side chains (e.g., alanine, valine, leucine, isoleucine, proline, phenylalanine, methionine), β-branched side chains (e.g., threonine, valine, isoleucine), and aromatic side chains (e.g., tyrosine, phenylalanine, tryptophan, histidine). Thus, it is preferred to replace a corresponding amino acid residue with another amino acid residue from the same side chain family. Methods for identifying conservative substitutions of amino acids are well known in the art (see, for example, Brummell et al., Biochem. 32: 1180 to 1187 (1993); Kobayashi et al. Protein Eng. 12(10): 879 to 884 (1999); and Burks et al. Proc. Natl Acad. Set USA 94: 412 to 417 (1997), which are incorporated herein by reference).

The twenty conventional amino acids referred to herein are written in accordance with conventional usage. See, for example, Immunology to A Synthesis (2nd Edition, E. S. Golub and D. R. Gren, Eds., Sinauer Associates, Sunderland, Mass. (1991)), which is incorporated herein by reference. In the present invention, the terms "polypeptide" and "protein" have the same meaning and are used interchangeably. And in the present invention, amino acids are generally represented by single-letter and three-letter abbreviations known in the art. For example, alanine can be represented by A or Ala.

### Beneficial effects of the invention

The fusion protein of the present application comprises RSV F1 truncate and F2 truncate in a unique truncation manner. The truncated fusion protein has the ability to induce a specific antibody comparable to the full-length pre-F protein, and can also induce a neutralization antibody titer significantly higher than that of the full-length pre-F protein. The presence of the linker contained in the fusion protein also plays a role in improving the stability of the pre-F specific epitope site Φ. Further, the fusion protein obtained above is mutated, and the mutated fusion protein has stronger affinity with the antibody, higher thermal stability, and stronger immunogenicity.

In summary, the fusion protein of the present application shows good protection and safety, and is suitable for various forms of vaccine platforms, such as nucleic acid vaccines, recombinant protein vaccines, viral vector vaccines, and granulated vaccines. Therefore, the fusion protein of the present application has great potential in inducing an immune response to RSV in a subject, and preventing and/or treating a RSV infection or a disease and/or symptom caused by RSV infection.

The embodiments of the present invention will be described in detail below in conjunction with the drawings and examples, but those skilled in the art will understand that the following drawings and examples are only used to illustrate the present invention, rather than to limit the scope of the present invention. According to the following detailed description of the drawings and preferred embodiments, the various objects and advantages of the present invention will become apparent to those skilled in the art.

### Brief Description of the Drawings

Fig. 1 shows the expression results of high-neutralization antibody epitopes site II and site Ø after transfection of 293t cells with F proteins containing different linker peptides.
Fig. 2 shows a schematic diagram of the structure of wild-type F protein F_{wt} and F protein truncate LC2.
Fig. 3 shows a schematic diagram of the structure of F protein truncate LC2 predicted by AlphaFold 2.
Fig. 4 shows the expression of F protein truncate detected by the Western blot, the first lane is Marker, the second lane is pre-F protein SC-TM, and the third lane is F protein truncate LC2.
Fig. 5 shows the binding of F protein truncate LC2 to site II specific antibody mota (Panel A in Fig. 5) and site Ø specific antibodies D25 and AM22 (Panel B and Panel C in Fig. 5) detected by immunofluorescence.
Fig. 6 shows the serum binding antibody titer and serum antibody typing in the mice induced by mRNA vaccine encoding F protein truncate.
Fig. 7 shows the results of neutralization antibody titer in the mice induced by mRNA vaccine encoding F protein truncate.
Fig. 8 shows the schematic diagram of the construction of wild-type F protein F_{wt} and mutated F protein truncate LC2A, in which the wild-type F protein F_{wt} comprises a signal peptide, F2, F1, a membrane anchoring region and a cytoplasmic region; the mutated F protein truncate LC2A comprises a signal peptide, a first linker peptide, a mutated first truncate, a linker, a mutated second truncate, a second linker peptide, and a multimerization motif.
Fig. 9 shows the results of polyacrylamide gel electrophoresis of the control protein SC-TM and the mutated F protein truncate, wherein the first lane is Marker, the second lane is LC2A, and the third lane is SC-TM.
Fig. 10 shows the binding of SC-TM and 7 mutated F protein truncates to three antibodies against F protein.
Fig. 11 shows the results of affinity of SC-TM and 7 mutated F protein truncates with two antibodies against F protein detected by Biacore, the results of protein thermal stability detected by DSF, and the results of epitope thermal stability detected by ELISA.
Fig. 12 shows the antibody titers in the serum of mice immunized with SC-TM and 7 mutated F protein truncates, wherein Panel A in Fig. 12 shows the antibody titers of binding to pre-F, and Panel B in Fig. 12 shows the antibody titers of binding to post-F.

### Sequence information

The information of some sequences involved in the present invention is provided in Table 1 below.

**Table 1: Description of sequence**

| SEQ ID NO: | Description | Sequence |
|---|---|---|
| 1 | Amino acid sequence of F_{wt} | MELLILKANAITTILTAVTFCFASGQNITEEFYQSTCSAVSKGYLSALR TGWYTSVITIELSNIKKNKCNGTDAKVKLIKQELDKYKNAVTELQLL MQSTQATNNRARRELPRFMNYTLNNAKKTNVTLSKKRKRRFLGFLL GVGSAIASGVAVSKVLHLEGEVNKIKSALLSTNKAVVSLSNGVSVLT SKVLDLKNYIDKQLLPIVNKQSCSISNIETVIEFQQKNNRLLEITREFSV NAGVTTPVSTYMLTNSELLSLINDMPITNDQKKLMSNNVQIVRQQSY SIMSIIKEEVLAYVVQLPLYGVIDTPCWKLHTSPLCTTNTKEGSNICLT RTDRGWYCDNAGSVSFFPQAETCKVQSNRVFCDTMNSLTLPSEVNL CNVDIFNPKYDCKIMTSKTDVSSSVITSLGAIVSCYGKTKCTASNKNR GIIKTFSNGCDYVSNKGVDTVSVGNTLYYVNKQEGKSLYVKGEPIIN FYDPLVFPSDEFDASISQVNEKINQSLAFIRKSDELLHNVNAGKSTTNI MITTIIIVIIVILLSLIAVGLLLYCKARSTPVTLSKDQLSGINNIAFSN |
| 2 | Linker peptide LC | GGGPGG |
| 3 | Amino acid sequence of LC1 | SAVSKGYLSALRTGWYTSVITIELSNIKKIKCNGTDAKIKLIKQELDKY KNAVTELQLLMQSTPATNNGGGPGGSAIASGVAVSKVLHLEGEVNK IKSALLSTNKAVVSLSNGVSVLTSKVLDLKNYIDKQLLPIVNKQSCSIP NIETVIEFQQKNNRLLEITREFSVNAGVTTPVSTYMLTNSELLSLINDM PITNDQKKLMSNNVQIVRQQSYSIMSIIKEEVLAYVVQLPLYGVIDTP CWKLHTSPLC |
| 4 | Amino acid sequence of LC2 | GWYTSVITIELSNIKENKCNGTDAKVKLIKQELDKYKNAVTELQLLM QSTPATNNGGGPGGSAIASGVAVSKVLHLEGEVNKIKSALLSTNKAV VSLSNGVSVLTSKVLDLKNYIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEVLAYVVQLPLY |
| 5 | Amino acid sequence of LC3 | VITIELSNIKENKCNGTDAKVKLIKQELDKYKNAVTELQLLMQSTPAT NNGGGPGGSAIASGVAVSKVLHLEGEVNKIKSALLSTNKAVVSLSNG VSVLTSKVLDLKNYIDKQLLPIVNKQSCSISNIETVIEFQQKNNRLLEIT REFSVNAGVTTPVSTYMLTNSELLSLINDMPITNDQKKLMSNNVQIV RQQSYSIMSIIKEEVLAYVVQLPLY |
| 6 | Amino acid sequence of LC4 | GWYTSVITIELSNIKENKCNGTDAKVKLIKQELDKYKNAVTELQLLM QSTPATNNGGGPGGSAIASGVAVSKVLHLEGEVNKIKSALLSTNKAV VSLSNGVSVLTSKVLDLKNYIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEVL |
| 7 | Amino acid sequence of LC5 | VITIELSNIKENKCNGTDAKVKLIKQELDKYKNAVTELQLLMQSTPAT NNGGGPGGSAIASGVAVSKVLHLEGEVNKIKSALLSTNKAVVSLSNG VSVLTSKVLDLKNYIDKQLLPIVNKQSCSISNIETVIEFQQKNNRLLEIT REFSVNAGVTTPVSTYMLTNSELLSLINDMPITNDQKKLMSNNVQIV RQQSYSIMSIIKEEVLG |
| 8 | Membrane-anchoring sequence | IMITTIIIVIIVILLSLIAVGLLLYC |
| 9 | Signal peptide | MDAMKRGLCCVLLLCGAVFVSA |
| 10 | pre-F protein | ATMELLILKANAITTILTAVTFCFASGQNITEEFYQSTCSAVSKGYLSA LRTGWYTSVITIELSNIKKIKCNGTDAKIKLIKQELDKYKNAVTELQL LMQSTPATNNQARGSGSGRSLGFLLGVGSAIASGVAVSKVLHLEGEV NKIKSALLSTNKAVVSLSNGVSVLTSKVLDLKNYIDKQLLPIVNKQSC SIPNIETVIEFQQKNNRLLEITREFSVNAGVTTPVSTYMLTNSELLSLIN DMPITNDQKKLMSNNVQIVRQQSYSIMSIIKEEVLAYVVQLPLYGVID TPCWKLHTSPLCTTNTKEGSNICLTRTDRGWYCDNAGSVSFFPQAET CKVQSNRVFCDTMNSLTLPSEVNLCNVDIFNPKYDCKIMTSKTDVSS SVITSLGAIVSCYGKTKCTASNKNRGIIKTFSNGCDYVSNKGVDTVSV GNTLYYVNKQEGKSLYVKGEPIINFYDPLVFPSDQFDASISQVNEKIN QSLAFIRKSDELLSAIGGYIPEAPRDGQAYVRKDGEWVLLSTFLGGIE GRHHHHHHSAWSHPQFEK |
| 11 | post-F protein | MELLILKANAITTILTAVTFCFASGQNITEEFYQSTCSAVSKGYLSALR TGWYTSVITIELSNIKENKCNGTDAKVKLIKQELDKYKNAVTELQLL MQSTPATNNRARRELPRFMNYTLNNAKKTNVTLSKKRKRRAIASGV AVSKVLHLEGEVNKIKSALLSTNKAVVSLSNGVSVLTSKVLDLKNYI DKQLLPIVNKQSCSISNIETVIEFQQKNNRLLEITREFSVNAGVTTPVST YMLTNSELLSLINDMPITNDQKKLMSNNVQIVRQQSYSIMSIIKEEVL AYVVQLPLYGVIDTPCWKLHTSPLCTTNTKEGSNICLTRTDRGWYCD NAGSVSFFPQAETCKVQSNRVFCDTMNSLTLPSEVNLCNVDIFNPKY DCKIMTSKTDVSSSVITSLGAIVSCYGKTKCTASNKNRGIIKTFSNGCD YVSNKGVDTVSVGNTLYYVNKQEGKSLYVKGEPIINFYDPLVFPSDE FDASISQVNEKINQSLAFIRKSDELLGLEVLFQGPHHHHHHHHSAWS HPQFEK |
| 12 | F1 | FLGFLLGVGSAIASGVAVSKVLHLEGEVNKIKSALLSTNKAVVSLSN GVSVLTSKVLDLKNYIDKQLLPIVNKQSCSISNIETVIEFQQKNNRLLE ITREFSVNAGVTTPVSTYMLTNSELLSLINDMPITNDQKKLMSNNVQI VRQQSYSIMSIIKEEVLAYVVQLPLYGVIDTPCWKLHTSPLCTTNTKE GSNICLTRTDRGWYCDNAGSVSFFPQAETCKVQSNRVFCDTMNSLTL PSEVNLCNVDIFNPKYDCKIMTSKTDVSSSVITSLGAIVSCYGKTKCT ASNKNRGIIKTFSNGCDYVSNKGVDTVSVGNTLYYVNKQEGKSLYV KGEPIINFYDPLVFPSDEFDASISQVNEKINQSLAFIRKSDELLHNVNA GKSTTNIMITTIIIVIIVILLSLIAVGLLLYCKARSTPVTLSKDQLSGINNI AFSN |
| 13 | F2 | QNITEEFYQSTCSAVSKGYLSALRTGWYTSVITIELSNIKKNKCNGTD AKVKLIKQELDKYKNAVTELQLLMQSTQATNNRARR |
| 14 | LC1 F1 truncate | SAIASGVAVSKVLHLEGEVNKIKSALLSTNKAVVSLSNGVSVLTSKV LDLKNYIDKQLLPIVNKQSCSISNIETVIEFQQKNNRLLEITREFSVNA GVTTPVSTYMLTNSELLSLINDMPITNDQKKLMSNNVQIVRQQSYSI MSIIKEEVLAYVVQLPLYGVIDTPCWKLHTSPLC |
| 15 | LC2 F1 truncate | SAIASGVAVSKVLHLEGEVNKIKSALLSTNKAVVSLSNGVSVLTSKV LDLKNYIDKQLLPIVNKQSCSISNIETVIEFQQKNNRLLEITREFSVNA GVTTPVSTYMLTNSELLSLINDMPITNDQKKLMSNNVQIVRQQSYSI MSIIKEEVLAYVVQLPLY |
| 16 | LC1 F2 truncate | EFYQSTCSAVSKGYLSALRTGWYTSVITIELSNIKKNKCNGTDAKVK LIKQELDKYKNAVTELQLLMQSTQATNN |
| 17 | LC2 F2 truncate | GWYTSVITIELSNIKKNKCNGTDAKVKLIKQELDKYKNAVTELQLLM QSTQATNN |
| 18 | Linker peptide | GGGSGG |
| 19 | LC1 fusion protein | MDAMKRGLCCVLLLCGAVFVSAGGGSGGSAVSKGYLSALRTGWYT SVITIELSNIKKIKCNGTDAKIKLIKQELDKYKNAVTELQLLMQSTPAT NNGGGPGGSAIASGVAVSKVLHLEGEVNKIKSALLSTNKAVVSLSNG VSVLTSKVLDLKNYIDKQLLPIVNKQSCSIPNIETVIEFQQKNNRLLEIT REFSVNAGVTTPVSTYMLTNSELLSLINDMPITNDQKKLMSNNVQIV RQQSYSIMSIIKEEVLAYVVQLPLYGVIDTPCWKLHTSPLCGGSGGSG GIMITTIIIVIIVILLSLIAVGLLLYC |
| 20 | LC2 fusion protein | MDAMKRGLCCVLLLCGAVFVSAGGGSGGGWYTSVITIELSNIKENK CNGTDAKVKLIKQELDKYKNAVTELQLLMQSTPATNNGGGPGGSAI ASGVAVSKVLHLEGEVNKIKSALLSTNKAVVSLSNGVSVLTSKVLDL KNYIDKQLLPIVNKQSCSISNIETVIEFQQKNNRLLEITREFSVNAGVT TPVSTYMLTNSELLSLINDMPITNDQKKLMSNNVQIVRQQSYSIMSIIK EEVLAYVVQLPLYGGSGGSGGIMITTIIIVIIVILLSLIAVGLLLYC |
| 21 | Trimerization motif Foldon | GYIPEAPRDGQAYVRKDGEWVLLSTFLGS |
| 22 | Dimerization motif mouse IgG2a constant region | TTAPSVYPLAPVCGGTTGSSVTLGCLVKGYFPEPVTLTWNSGSLSSG VHTFPALLQSGLYTLSSSVTVTSNTWPSQTITCNVAHPASSTKVDKKI EPRVPITQNPCPPLKECPPCAAPDLLGGPSVFIFPPKIKDVLMISLSPMV TCVVVDVSEDDPDVQISWFVNNVEVHTAQTQTHREDYNSTLRVVSA LPIQHQDWMSGKAFACAVNNRALPSPIEKTISKPRGPVRAPQVYVLP PPAEEMTKKEFSLTCMITGFLPAEIAVDWTSNGRTEQNYKNTATVLD SDGSYFMYSKLRVQKSTWERGSLFACSVVHEGLHNHLTTKTISRSLG KGG |
| 23 | Linker peptide LA | GGGSGP |
| 24 | Linker peptide LB | GGSG |
| 25 | Linker peptide LD | GGGSGGGSPG |
| 26 | Linker peptide LE | EAAAK |
| 27 | Linker peptide LF | PAAK |
| 28 | Amino acid sequence of LC2A | GWYTSVITIELSNIKENKCNGTDAKVKLLKQELDKYKNAVTELQLLM QSTPATNNGGGPGGSAIASGVAVSKVLHLEGEVNKIKSALLSTNKAV VSLSNGVSVLTSKVLDLKNYIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEMLAYVVQLPLY |
| 29 | Amino acid sequence of LC2B | GWYTSVITIELSNIKENKCNGTDAKVKLLKQELDKYKNAVTELQLLM QSTPATNNGGGPGGSAIASGVAVSKVLHLEGEVNKIKSALLSTNKAV VSLSNGVSVLTSKVLDLKNYIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEVLLYVVQLPLY |
| 30 | Amino acid sequence of LC2C | GWYTSVITIELSNIKENKCNGTDAKVKLLKQELDKYKNAVTELQLLM QSTPATNNGGGPGGSAIASGVAVSKVLHLEGEVNKIKSALLSTNKAI VSLSNGVSVLTSKVLDLKNYIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEVLAYVVQLPLY |
| 31 | Amino acid sequence of LC2D | GWYTSVITIELSNIKENKCNGTDAKVKLIKQELDKYKNAVTELQLLA QSTPATNNGGGPGGSAIASGVAVSKVLHLEGEVNKIKSALLSTNKAV VSLSNGVSVLTSKVLDLKNIIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEVLAYVVQLPLY |
| 32 | Amino acid sequence of LC2E | GWYTSVITIELSNIKENKCNGTDAKVKLLKQELDKYKNAVTELQLLA QSTPATNNGGGPGGSAIASGVAVSKVLHLEGEVNKIKSALLSTNKAV VSLSNGISVLTSKVLDLKNYIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEVLLYVVQLPLY |
| 33 | Amino acid sequence of LC2F | GWYTSVITIELSNIKENKCNGTDAKVKLIKQELDKYKNAVTELQLLM QSTPATNNGGGPGGSAIASGVAVSKVLHLEGEVNKIKSALASTNKAV VSLSNGISVLTSKVLDLKNIIDKQLLPIVNKQSCSISNIETVIEFQQKNN RLLEITREFSVNAGVTTPVSTYMLTNSELLSLINDMPITNDQKKLMSN NVQIVRQQSYSIMSIIKEEVLLYVVQLPLY |
| 34 | Amino acid sequence of LC2G | GWYTSVITIELSNIKENKCNGTDAKVKLIKQELDKYKNAVTELQLLM QSTPATNNGGGPGGSAIASGVAVSKVLHLEGEVNKIKSALLSTNKAI VSLSNGVSVLTSKVLDLKNIIDKQLLPIVNKQSCSISNIETVIEFQQKN NRLLEITREFSVNAGVTTPVSTYMLTNSELLSLINDMPITNDQKKLMS NNVQIVRQQSYSIMSIIKEEVLAYVVQLPLY |
| 35 | Amino acid sequence of SC-TM | MELLILKANAITTILTAVTFCFASGQNITEEFYQSTCSAVSKGYLSALR TGWYTSVITIELSNIKKIKCNGTDAKIKLIKQELDKYKNAVTELQLLM QSTPATNNQARGSGSGRSLGFLLGVGSAIASGVAVSKVLHLEGEVNK IKSALLSTNKAVVSLSNGVSVLTSKVLDLKNYIDKQLLPIVNKQSCSIP NIETVIEFQQKNNRLLEITREFSVNAGVTTPVSTYMLTNSELLSLINDM PITNDQKKLMSNNVQIVRQQSYSIMSIIKEEVLAYVVQLPLYGVIDTP CWKLHTSPLCTTNTKEGSNICLTRTDRGWYCDNAGSVSFFPQAETCK VQSNRVFCDTMNSLTLPSEVNLCNVDIFNPKYDCKIMTSKTDVSSSVI TSLGAIVSCYGKTKCTASNKNRGIIKTFSNGCDYVSNKGVDTVSVGN TLYYVNKQEGKSLYVKGEPIINFYDPLVFPSDQFDASISQVNEKINQS LAFIRKSDELLSAIGGYIPEAPRDGQAYVRKDGEWVLLSTFLGGIEGR HHHHHH |
| 36 | MOTA VH | QVTLRESGPALVKPTQTLTLTCTFSGFSLSTAGMSVGWIRQPPGKALE WLADIWWDDKKHYNPSLKDRLTISKDTSKNQVVLKVTNMDPADTA TYYCARDMIFNFYFDVWGQGTTVTVSSASTKGPSVFPLAPSSKSTSG GTAALGCLVKDYFPEPVTVSWNSGALTSGVHTFPAVLQSSGLYSLSS VVTVPSSSLGTQTYICNVNHKPSNTKVDKKVEPKSCDK |
| 37 | MOTA VL | DIQMTQSPSTLSASVGDRVTITCSASSRVGYMHWYQQKPGKAPKLLI YDTSKLASGVPSRFSGSGSGTEFTLTISSLQPDDFATYYCFQGSGYPFT FGGGTKVEIKRTVAAPSVFIFPPSDEQLKSGTASVVCLLNNFYPREAK VQWKVDNALQSGNSQESVTEQDSKDSTYSLSSTLTLSKADYEKHKV YACEVTHQGLSSPVTKSFNRGEC |
| 38 | D25 VH | QVQLVQSGAEVKKPGSSVMVSCQASGGPLRNYIINWLRQAPGQGPE WMGGIIPVLGTVHYAPKFQGRVTITADESTDTAYIHLISLRSEDTAMY YCATETALVVSTTYLPHYFDNWGQGTLVTVSSASTKGPSVFPLAPSS KSTSGGTAALGCLVKDYFPEPVTVSWNSGALTSGVHTFPAVLQSSGL YSLSSVVTVPSSSLGTQTYICNVNHKPSNTKVDKKVEPKSCDK |
| 39 | D25 VL | DIQMTQSPSSLSAAVGDRVTITCQASQDIVNYLNWYQQKPGKAPKLL IYVASNLETGVPSRFSGSGSGTDFSLTISSLQPEDVATYYCQQYDNLP LTFGGGTKVEIKRTVAAPSVFIFPPSDEQLKSGTASVVCLLNNFYPRE AKVQWKVDNALQSGNSQESVTEQDSKDSTYSLSSTLTLSKADYEKH KVYACEVTHQGLSSPVTKSFNRGEC |
| 40 | hRSV90 VH | EVQLVESGGGLVQPRRSLRLSCAASGFTFDDYTIHWVRQAPGKGLE WVSGITWNSGYIGYADSVKGRFTISRDNARNSLYLQMNSLRAEDTA LYYCVRDAYVSGSDYYYYGLDVWGRGTLVTVSSASTKGPSVFPLAP SSKSTSGGTAALGCLVKDYFPEPVTVSWNSGALTSGVHTFPAVLQSS GLYSLSSVVTVPSSSLGTQTYICNVNHKPSNTKVDKKVEP |
| 41 | hRSV90 VL | EIVMTSSPATLSVSPGERVTLFCRASQSVISNLAWYQQKSGQAPRLLI YGASTRATGIPSRFSGSGSGTEFTLTISSLQSEDFAVYFCQQYNNWPL TFGGGTQVNVQRTVAAPSVFIFPPSDEQLKSGTASVVCLLNNFYPRE AKVQWKVDNALQSGNSQESVTEQDSKDSTYSLSSTLTLSKADYEKH KVYACEVTHQGLSSPVTKSFNRGEC |
| 42 | Human antibody heavy chain constant region amino acid sequence | |
| 43 | Human antibody light chain constant region amino acid sequence | |
| 44 | Amino acid sequence of post-F | MELLILKANAITTILTAVTFCFASGQNITEEFYQSTCSAVSKGYLSALR TGWYTSVITIELSNIKENKCNGTDAKVKLIKQELDKYKNAVTELQLL MQSTPATNNRARRELPRFMNYTLNNAKKTNVTLSKKRKRRAIASGV AVSKVLHLEGEVNKIKSALLSTNKAVVSLSNGVSVLTSKVLDLKNYI DKQLLPIVNKQSCSISNIETVIEFQQKNNRLLEITREFSVNAGVTTPVST YMLTNSELLSLINDMPITNDQKKLMSNNVQIVRQQSYSIMSIIKEEVL AYVVQLPLYGVIDTPCWKLHTSPLCTTNTKEGSNICLTRTDRGWYCD NAGSVSFFPQAETCKVQSNRVFCDTMNSLTLPSEVNLCNVDIFNPKY DCKIMTSKTDVSSSVITSLGAIVSCYGKTKCTASNKNRGIIKTFSNGCD YVSNKGVDTVSVGNTLYYVNKQEGKSLYVKGEPIINFYDPLVFPSDE FDASISQVNEKINQSLAFIRKSDELLGLEVLFQGPHHHHHHHHSAWS HPQFEK |
| 45 | Fusion protein of LC2A | MDAMKRGLCCVLLLCGAVFVSAGGGSGGGWYTSVITIELSNIKENK CNGTDAKVKLLKQELDKYKNAVTELQLLMQSTPATNNGGGPGGSAI ASGVAVSKVLHLEGEVNKIKSALLSTNKAVVSLSNGVSVLTSKVLDL KNYIDKQLLPIVNKQSCSISNIETVIEFQQKNNRLLEITREFSVNAGVT TPVSTYMLTNSELLSLINDMPITNDQKKLMSNNVQIVRQQSYSIMSIIK EEMLAYVVQLPLYGGGSGGGYIPEAPRDGQAYVRKDGEWVLLSTFL GSHHHHHHH |
| 46 | Fusion protein of LC2B | MDAMKRGLCCVLLLCGAVFVSAGGGSGGGWYTSVITIELSNIKENK CNGTDAKVKLLKQELDKYKNAVTELQLLMQSTPATNNGGGPGGSAI ASGVAVSKVLHLEGEVNKIKSALLSTNKAVVSLSNGVSVLTSKVLDL KNYIDKQLLPIVNKQSCSISNIETVIEFQQKNNRLLEITREFSVNAGVT TPVSTYMLTNSELLSLINDMPITNDQKKLMSNNVQIVRQQSYSIMSIIK EEVLLYVVQLPLYGGGSGGGYIPEAPRDGQAYVRKDGEWVLLSTFL GSHHHHHHH |

### Specific Models for Carrying Out the present invention

The present invention is now described with reference to the following examples which are intended to illustrate the present invention (but not to limit the present invention).

Unless otherwise specified, the experiments and methods described in the examples were basically carried out according to conventional methods well known in the art and described in various references. For example, conventional techniques of immunology, biochemistry, chemistry, molecular biology, microbiology, cell biology, genomics and recombinant DNA used in the present invention could be found in Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel et al., eds., (1987)); METHODS IN ENZYMOLOGY series (Academic Publishing Company): PCR 2: A PRACTICAL METHOD. APPROACH) (M.J. MacPherson, B.D. Hames and G.R. Taylor, ed. (1995)), and ANIMAL CELL CULTURE (R.I. Freshney, ed. (1987)).

In addition, if the specific conditions were not specified in the examples, they were carried out according to conventional conditions or the conditions recommended by the manufacturer. The reagents or instruments used without indicating the manufacturer were all conventional products that could be obtained commercially. It is known to those skilled in the art that the examples describe the present invention by way of example and are not intended to limit the scope of protection as claimed in the present invention. All the disclosures and other references mentioned herein are incorporated herein by reference in their entirety.

### Example 1. Screening experiment of flexible linker peptides of F protein truncates

During the conformational change of RSV F protein, the release of the fusion peptide in the hydrophobic cavity is a very critical triggering event. In order to help the fusion peptide keep stable in the hydrophobic cavity to stabilize the pre-F conformation, flexible linker peptides of different lengths were introduced at the C-terminal of F2 (SEQ ID NO: 12) and the N-terminal of F1 (SEQ ID NO: 11). A total of 6 flexible linker peptides were designed, and the amino acid sequences were set forth in SEQ ID NOs: 23, 2, 24, 25, 26 and 27, respectively. The nucleotide sequences of the F proteins containing the above flexible linker peptides were submitted to the company (Shanghai Shenggong Biotechnology Co., Ltd.) for humanized codon optimization, underwent introduction with HindIII and xBaI restriction sites at the 5' and 3' ends, respectively, and inserted into the pcDNA3.1 eukaryotic expression vector. F protein recombinant plasmids containing the flexible linker peptides were constructed, which were named LA, LB, LC, LD, LE and LF, respectively. Immunofluorescence was used to detect the expression of high-neutralization antibody epitopes site II and site Ø of the F proteins containing the flexible linker peptides.
1) On the first night, 293T cells were inoculated into a 96-well plate at 3×10⁴/well.
2) On the next day, when the cell confluence reached 85%, plasmid transfection was performed. 5µL of opti-MEM culture medium was taken and added to a 96-well U-bottom plate, added with 0.2µg of plasmid and 0.3µL of P3000, vortexed and mixed, and marked as A. Another 5µL of opti-MEM culture medium was taken and added to a 96-well U-bottom plate, added with 0.3µL of Lipofectamine 3000, vortexed and mixed, and marked as B. The mixture in Tube A was added to tube B, vortexed and mixed, and allowed to stand for 15 minutes. Then the solution was added to the plate coated with 293t cells, shaken gently and placed in a cell culture incubator for culture.
3) After transfection was performed for 24 hours, 100µL of 4% formaldehyde solution was added to each well to fix the cells at room temperature for 30 minutes.
4) The cell culture medium and the formaldehyde solution were discarded, and then the cells were washed 3 times with PBS, 5 minutes each time.
5) 100µL of PBS containing 2% skim milk powder was added to each well to perform blocking at room temperature for 2 hours.
6) After washing was performed once with PBS, antibodies capable of recognizing different epitopes (site II: motavizumab, site Ø: AM22, D25) were added respectively, and incubated at room temperature for 1 hour.
7) Washing was performed 3 times with PBS.
8) 100µL of Alexa Fluor 568 (1: 2000 dilution) (Manufacturer: Thermo; Cat. No.: A-11013) fluorescent secondary antibody was added to each well, and incubated at room temperature for 1 hour.
9) Washing was performed 3 times with PBS.
10) 100µL of nuclear dye DAPI (Manufacturer: Invitrogen; Cat. No.: D1306) was added to each well to perform staining for 10 minutes.
11) Washing was performed 3 times with PBS.
12) Pictures were taken and quantitative analysis was performed with a high-content imaging system.

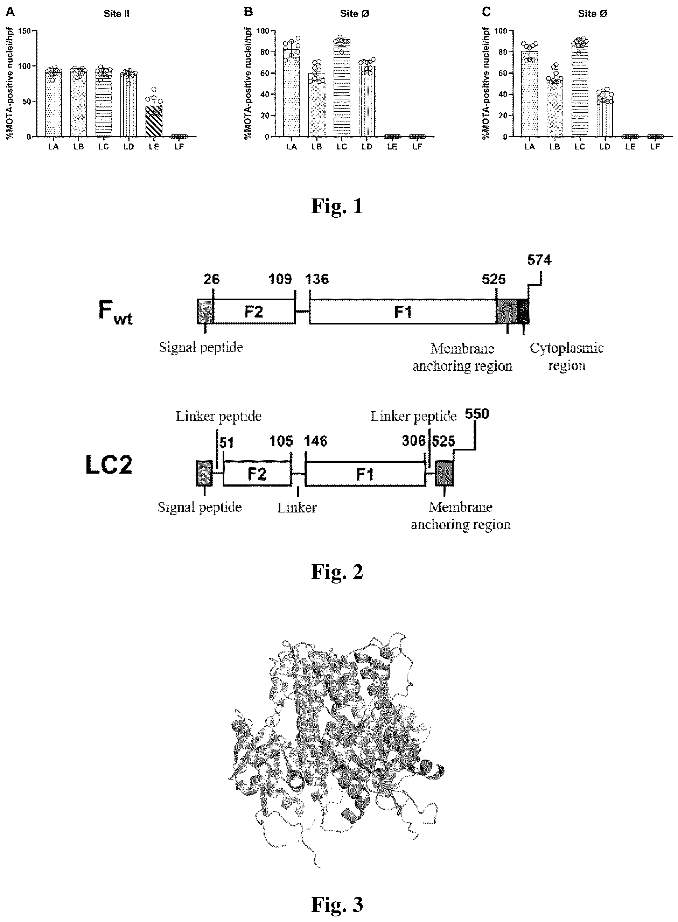

Fig. 1 showed the expression results of high-neutralization antibody epitopes site II and site Ø after transfection of 293t cells with F proteins containing different linker peptides. The results showed that the F proteins containing flexible linker peptides LA and LC retained the highest expression of site Ø.

### Example 2. Design of F protein truncate

The amino acid sequence of RSV A2 Fusion protein was obtained from the GenBank database (GenBank ID: FJ614814.1), and the amino acid sequence of F_{wt} was set forth in SEQ ID NO: 1. The amino acid sequence characteristics of the F protein truncate were that the N-terminal and C-terminal of the F2 peptide chain and the F1 peptide chain were truncated to obtain a truncated F2 and a truncated F1, and the truncated F2 was linked to the truncated F1 through a flexible linker peptide containing proline to help stabilize the pre-F conformation. The C-terminal of the truncated F1 was linked to different display motifs through flexible linker peptides. A total of 10 F protein truncates were prepared, which were as follows:
(1) The amino acids at positions 31 to 105 and amino acids at positions 145 to 322 of SEQ ID NO: 1 were linked through a flexible linker peptide containing proline (SEQ ID NO: 2). The F protein truncate had a total length of 314 amino acids and was named LC1, and its amino acid sequence was set forth in SEQ ID NO: 3. LC1 retained the epitopes at site Ø, site V, site III and site II.
(2) The amino acids at positions 51 to 105 and amino acids at positions 145 to 306 of SEQ ID NO: 1 were linked through a flexible linker peptide containing proline (SEQ ID NO: 2). The F protein truncate had a total length of 284 amino acids and was named LC2, and its amino acid sequence was set forth in SEQ ID NO: 4. LC2 retained the epitopes at site Ø, site V and site II.
(3) The amino acids at positions 56 to 105 and amino acids at positions 145 to 306 of SEQ ID NO: 1 were linked through a flexible linker peptide containing proline (SEQ ID NO: 2). The F protein truncate had a total length of 280 amino acids and was named LC3, and its amino acid sequence was set forth in SEQ ID NO: 5. LC3 retained the epitopes at site Ø and site II.
(4) The amino acids at positions 51 to 105 and amino acids at positions 145 to 297 of SEQ ID NO: 1 were linked through a flexible linker peptide containing proline (SEQ ID NO: 2). The F protein truncate had a total length of 276 amino acids and was named LC4, and its amino acid sequence was set forth in SEQ ID NO: 6. LC4 retained the epitopes at site Ø and site II.
(5) The amino acids at positions 56 to 105 and amino acids at positions 145 to 297 of SEQ ID NO: 1 were linked through a flexible linker peptide containing proline (SEQ ID NO: 2). The F protein truncate had a total length of 271 amino acids and was named LC5, and its amino acid sequence was set forth in SEQ ID NO: 7. LC5 retained the epitope at site II.

### Example 3. Construction of recombinant plasmids expressing F protein truncates

The nucleotide sequences encoding the five F protein truncates were separately linked to the nucleotide sequence encoding the F protein cytoplasmic segment (amino acid sequence was set forth in SEQ ID NO: 8) through the nucleotide sequence encoding a flexible linker peptide. The nucleotide sequence encoding a signal peptide (amino acid sequence was set forth in SEQ ID NO: 9) was introduced at the 5' end of each of the nucleotide sequences of the above truncates. The nucleotide sequences of the F protein truncates were submitted to the company (Shanghai Shenggong Biotechnology Co., Ltd.) for humanized codon optimization, subjected to the introduction with HindIII and xBaI restriction sites at the 5' and 3' ends, respectively, and were inserted into the pcDNA3.1 eukaryotic expression vector to construct recombinant plasmids containing the F protein truncate target genes. Fig. 2 showed a schematic diagram of the connection of the F protein truncate LC2. Fig. 3 showed a schematic diagram of the structure of the F protein truncate predicted by AlphaFold 2. In addition, the full-length F protein SC-TM recombinant plasmid with the pre-F structure was constructed as a control according to the above method (the sequence of SC-TM protein was obtained from GenBank: 5C6B_F), and was synthesized by the company (Shanghai Shenggong Biotechnology Co., Ltd.). The successfully sequenced plasmids underwent large-scale plasmid extraction, followed by single digestion with the restriction enzyme XbaI. The single digestion results were identified by capillary electrophoresis. The identified plasmids were detected by spectrophotometer for OD260 and OD280, thereby determining their DNA concentrations and purity. The plasmids were stored at -20°C.

### Example 4: Detection of recombinant plasmid expression by Western Blot

1) On the first night, 293T cells were inoculated into a 6-well plate at 8×10⁵/well .
2) On the second day, when the cell confluence reached 85%, plasmid transfection was performed. 125µL of opti-MEM (manufacturer: Gibco; Cat. No.: 31985-070) culture medium was taken and added to a 1.5mL centrifuge tube, added with 2µg of plasmid, 4µL of P3000, vortexed and mixed, and marked as A. Another 125µL of opti-MEM culture medium was taken and added to another 1.5mL centrifuge tube, added with 4µL of Lipofectamine 3000 (manufacturer: Invitrogen; Cat. No.: L3000015), vortexed and mixed, and marked as B. The mixture in tube A was added to tube B, vortexed and mixed, and allowed to stand for 15 minutes. Then the solution was added to the 6-well plate with 293t cells, shaken gently, and placed in a cell culture incubator for culture.
3) On the third day, the culture medium was discarded, and pre-cooled PBS was added to wash the cells twice.
4) 150µL of RIPA lysis buffer containing protease inhibitor was added, and the cells were lysed on ice for 30 minutes.
5) Centrifugation was performed at 12000 rpm and 4°C for 20 minutes, and the supernatant was collected.
6) BCA quantitative reagent (manufacturer: PIERCE; Cat. No.: 23225) was used to determine the protein concentration of the supernatant.
7) The loading amount of sample for electrophoresis was 20µg/well, β-mercaptoethanol and loading buffer were added to the sample, and heat denaturation was performed at 100°C for 10 minutes.
8) A commercially available 12% precast gel was added, and electrophoresis was performed at a voltage of 180V for 40 minutes.
9) After electrophoresis, the protein was transferred to NC membrane using the wet transfer method.
10) Blocking was performed with PBS solution containing 5% skim milk powder for 2 hours.
11) Washing was performed once with PBST.
12) Human monoclonal antibody Motavizumab was diluted at 1: 2000 with 2% skim milk powder, and the NC membrane was transferred to the diluted antibody and incubated on a shaker at room temperature for 2 hours.
13) Washing was performed three times with PBST, 5 minutes each time.
14) GAH-HRP was diluted with PBS containing 2% skim milk powder, the NC membrane was transferred to the diluted secondary antibody, and incubated on a shaker at room temperature for 1 hour.
15) Washing was performed three times with PBST, 5 minutes each time.
16) The substrate for color development was added, pictures were taken and detection was performed with WB imaging system.

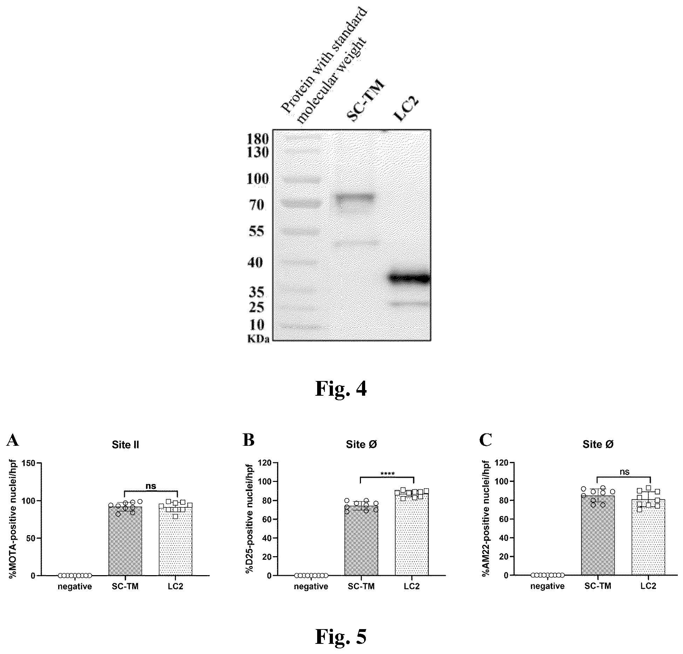

As shown in Fig. 4, the expression of F protein truncate on 293t cells was analyzed by Western Blot. The detection antibody was Motavizumab. The leftmost channel in the figure was a 180kD protein marker. SC-TM was the control pre-F protein, and LC2 was the F protein truncate described in the present invention.

### Example 5: Immunofluorescence detection of epitope integrity of F protein truncates

1) On the first night, 293T cells were inoculated into a 96-well plate at 3×10⁴/well.
2) On the next day, when the cell confluence reached 85%, plasmid transfection was performed. 5µL of opti-MEM culture medium was taken and added to a 96-well U-bottom plate, added with 0.2µg of plasmid and 0.3µL of P3000, vortexed and mixed, and marked as A. Another 5µL of opti-MEM culture medium was taken and added to a 96-well U-bottom plate, added with 0.3µL of Lipofectamine 3000, vortexed and mixed, and marked as B. The mixture in tube A was added to tube B, vortexed and mixed, and allowed to stand for 15 minutes. Then the solution was added to the plate with 293t cells, shaken gently and placed in a cell culture incubator for culture.
3) 24 hours after transfection, 100µL of 4% formaldehyde solution was added to each well to fix the cells at room temperature for 30 minutes.
4) The cell culture medium and the formaldehyde solution were discarded, and the cells were washed 3 times with PBS, 5 min each time.
5) 100µL of PBS containing 2% skim milk powder was added to each well, and blocking was performed at room temperature for 2 hours.
6) After washing once with PBS, antibodies capable of recognizing different epitopes (site II: motavizumab, site Ø: AM22, D25) were added respectively, and incubated at room temperature for 1 hour.
7) Washing was performed 3 times with PBS.
8) 100µL of Alexa Fluor568 (1: 2000 dilution) (Manufacturer: Thermo; Cat. No.: A-11013) fluorescent secondary antibody was added to each well and incubated at room temperature for 1 hour.
9) Washing was performed 3 times with PBS.
10) 100µL of nuclear dye DAPI (Manufacturer: Invitrogen; Cat. No.: D1306) was added to each well, and staining was performed for 10 minutes.
11) Washing was performed 3 times with PBS.
12) Pictures were taken and quantitative analysis was performed with high-content imaging system.

As shown in Fig. 5, immunofluorescence showed the expression of high-neutralization antibody epitopes site II and site Ø of full-length pre-F protein SC-TM and F protein truncate LC2 after transfection of 293t cells.

### Example 6. Construction of F protein truncate mRNA vaccine

According to the method described in the literature published by Andrew J Bett et al. (PMID: 32128257), RNA transcription-related elements were constructed in the in vitro transcription plasmid containing F protein truncate LC2 and full-length pre-F protein SC-TM, and plasmid extraction and identification were performed in consistent with those described in Example 1. After large-scale plasmid extraction, linearization was performed using restriction endonuclease BspQ1. Transcription was performed using the T7 in vitro transcription kit (manufacturer: Nanjing Nuoweizan Biotechnology Co., Ltd.; Cat. No.: DD4202) to obtain a capped mRNA. The transcription template was digested with DNase I. The mRNA was purified by precipitation using lithium chloride (manufacturer: Invitrogen; Cat. No.: AM9480).

The purified mRNA was dissolved in an acidic sodium citrate buffer for later use. Preparation of lipid nanoparticle LNP: cationic lipid: DSPC: cholesterol: DMG-PEG2000 at a molar ratio of 50: 10: 38.5: 1.5 were dissolved and mixed in ethanol. The mRNA and lipid mixed solution were encapsulated at a flow rate ratio of 3: 1. After encapsulation, it was dialyzed into PBS solution to obtain an mRNA vaccine.

### Example 7. Immunization of F protein truncate mRNA vaccine

The feeding and experimental operation of experimental animals were carried out in accordance with the procedures established by the Experimental Animal Use and Management Committee of Xiamen University. The experimental animals were BALB/c female mice purchased from Beijing Weitong Lihua Experimental Animal Technology Co., Ltd., 6 to 8 weeks old, and raised at the Experimental Animal Center of Xiamen University.
1) The purchased mice were randomly divided into groups, 4 mice in each group. The mRNA-LNP was injected at 5µg/mouse into the quadriceps femoris of the thigh, and PBS without mRNA-LNP was injected in the control group.
2) A second booster immunization was performed 2 weeks after the first immunization, a total of two immunizations.
3) Blood was collected from the eye sockets 14 days after booster immunization, and serum was separated for analysis.

### Example 8. Serum antibody titer and typing detection

1) The construction method of pre-F protein and post-F protein for coating was as follows: the sequences of pre-F and post-F proteins were downloaded from GenBank (GenBank ID: LY628284.1, GenBank ID: 6APB_A), the nucleotide sequences encoding the two proteins were cloned into the pcDNA3.1 eukaryotic expression vector, expressed using the CHO eukaryotic expression system, and purified by affinity chromatography to obtain the pre-F protein and post-F protein.
2) On the first night, the pre-F protein (SC-TM) and post-F protein were diluted with PBS, and coated on a 96-well plate at a dosage of 100 ng/well at 4°C overnight.
3) The solution for coating protein was discarded, and washing was performed once with PBST.
4) 5% fetal bovine serum blocking solution was added at 200 µL/well, and incubated at 37°C for 2 hours.
5) The blocking solution was discarded, and washing was performed once with PBST.
6) The gradiently diluted mouse serum (200 times in the first well, 5 times gradient) was added at 100 µL/well, and incubated at 37°C for 1 hour.
7) The serum dilution solution was discarded, and washing was performed five times with PBST.
8) Horseradish peroxidase-labeled goat anti-mouse IgG antibody, IgG1 antibody and IgG2a antibody (manufacturer: Abcam; Cat. No.: ab97265) diluted at 1: 5000 were added at 100 µL/well, respectively, and incubated at 37°C for 1 hour.
9) The secondary antibody was discarded, and washing was performed five times with PBST.
10) The color-development solution was added at 100 µL/well, and color-development was performed at room temperature in the dark for 10 minutes.
11) The stop solution was added at 50 µL/well, and the absorbance at 450 nm was detected with an ELISA reader.
12) The judgment standard for the endpoint titer was: 3 times the reading value of the negative well.

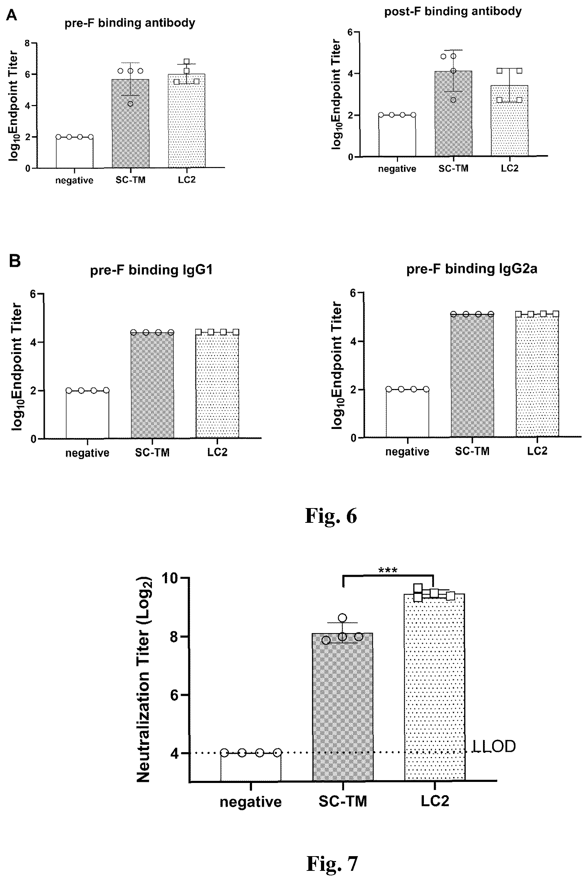

The experimental results were shown in Fig. 6. Panel A in Fig. 6 showed that the F protein truncate LC2 of the present application induced a high level of pre-F binding antibody titer, which was equivalent to that of SC-TM (i.e., full-length pre-F protein). Panel B in Fig. 6 showed that when immunization was performed in the form of mRNA vaccine, a higher level of IgG2a antibody was induced, indicating a Th-1 bias.

### Example 9. Detection of neutralization antibody titer

1) The serum separated after orbital blood collection was incubated at 56°C for 30 minutes to inactivate complement. 2) Gradient dilution of serum: 10 times dilution in the first well, and 4 times gradient dilution for a total of 10 gradients. 3) Isometric rRSV-mkatushka2 (MOI=0.1) was added, mixed and incubated at 37°C for 1 hour. 4) The mixture of serum and virus was transferred at a volume of 100 µL/well to a 96-well plate coated with a monolayer of Hela cells, and incubated at 37°C. 5) 24 hours after culture, the plate was read using the SpectraMax Paradigm Multi-Mode Microplate Reader.

The experimental results were shown in Fig. 7. The experimental results showed that as compared to SC-TM (i.e., full-length pre-F protein), the F protein truncate LC2 of the present application induced a neutralization antibody titer significantly higher than that of SC-TM.

In summary, compared to the full-length F protein, the F protein truncates of the example of the present invention had induced a higher level of pre-F specific antibody and a significantly higher neutralization antibody titer, showed good protection and safety, and were suitable for various forms of vaccine platforms, such as nucleic acid vaccine, recombinant protein vaccine, viral vector vaccine, and granulated vaccine.

### Example 10. Design of mutated F protein truncates

In this example, the F protein truncate LC2 (SEQ ID NO: 4) obtained in the above example was used as the basis, and further mutated to prepare a total of 7 mutants, which were specifically described as follows:
(1) In SEQ ID NO: 4, the amino acid I at position 29 was mutated to L, and the amino acid V at position 212 was mutated to M. The mutated F protein truncate was named LC2A, and its amino acid sequence was set forth in SEQ ID NO: 28.
(2) In SEQ ID NO: 4, the amino acid I at position 29 was mutated to L, and the amino acid A at position 214 was mutated to L. The mutated F protein truncate was named LC2B, and its amino acid sequence was set forth in SEQ ID NO: 29.
(3) In SEQ ID NO: 4, the amino acid I at position 29 was mutated to L, and the amino acid V at position 94 was mutated to I. The mutated F protein truncate was named LC2C, and its amino acid sequence was set forth in SEQ ID NO: 30.
(4) In SEQ ID NO: 4, the amino acid M at position 47 was mutated to A, and the amino acid Y at position 114 was mutated to I. The mutated F protein truncate was named LC2D, and its amino acid sequence was set forth in SEQ ID NO: 31.
(5) In SEQ ID NO: 4, the amino acid I at position 29 was mutated to L, the amino acid M at position 47 was mutated to A, the amino acid V at position 101 was mutated to I, and the amino acid A at position 214 was mutated to L. The mutated F protein truncate was named LC2E, and its amino acid sequence was set forth in SEQ ID NO: 32.
(6) In SEQ ID NO: 4, the amino acid L at position 88 was mutated to A, the amino acid V at position 101 was mutated to I, the amino acid Y at position 114 was mutated to I, and the amino acid A at position 214 was mutated to L. The mutated F protein truncate was named LC2F, and its amino acid sequence was set forth in SEQ ID NO: 33.
(7) In SEQ ID NO: 4, the amino acid V at position 94 was mutated to I, and the amino acid Y at position 114 was mutated to I. The mutated F protein truncate was named LC2G, and its amino acid sequence was set forth in SEQ ID NO: 34.

### Example 11. Construction of recombinant plasmids expressing F protein truncates

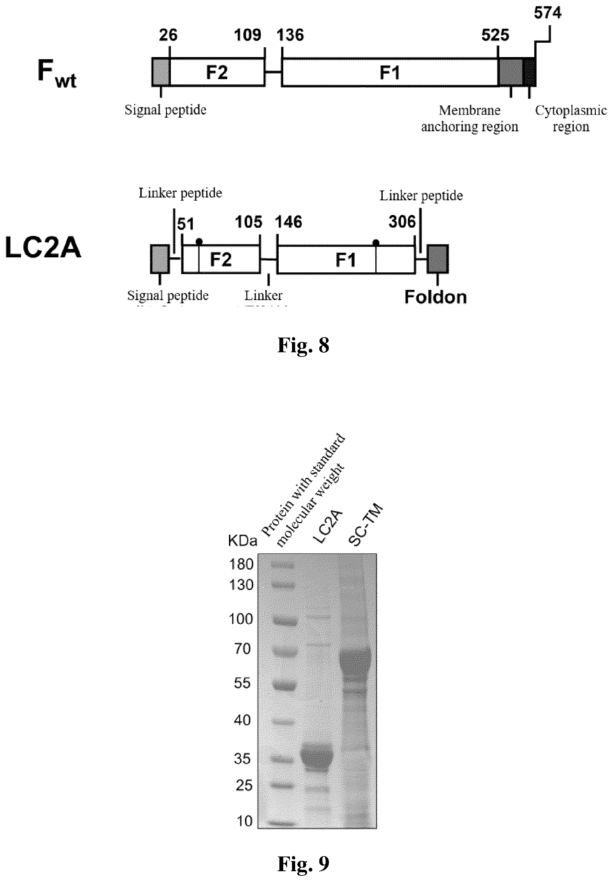

The nucleotide sequences encoding the 7 mutated F protein truncates were separately linked to the nucleotide sequence encoding a trimerization motif foldon (amino acid sequence was set forth in SEQ ID NO: 21) through the nucleotide sequence encoding GSGS amino acid; and, the nucleotide sequence encoding a signal peptide (SEQ ID NO: 9) was introduced at the 5' end of the nucleotide sequences encoding the 7 mutated F protein truncates to construct fusion expression genes. The nucleotide sequences of the mutated F protein truncates were submitted to Shanghai Shenggong Biotechnology Co., Ltd. for humanization codon optimization, underwent introduction with HindIII and xBaI restriction sites at the 5' end and 3' end respectively, and inserted into the pcDNA3.1 eukaryotic expression vector to construct recombinant plasmids containing the F protein truncate target genes. In addition, the full-length F protein SC-TM recombinant plasmid with the pre-Fusion structure was constructed as a control according to the above method (the sequence of SC-TM protein was obtained from GenBank: 5C6B_F, SEQ ID NO: 35), and synthesized by Shanghai Shenggong. Fig. 8 showed the schematic connection diagrams of the wild-type F protein F_{wt}, the control F protein SC-TM, and the mutated F protein truncate LC2A.

The plasmid powder with the target gene was dissolved in 10µL of double distilled water, and 1µL thereof was taken and added to 100µL of DH5α competent cells (*Escherichia coli*)*.* The sample was incubated on ice for 30 min, heat-shocked at 42 °C for 90 sec, and incubated on ice for 2 min. Then the *Escherichia coli* after reaction was added to LB medium, and coated on an agarose medium plate containing ampicillin resistance for culture. After the colonies grew, the monoclonal colonies were picked, and the plasmid was extracted with an endotoxin-free plasmid extraction kit (manufacturer: Tiangen; Cat. No.: DP117) for later use.

### Example 12. Expression and purification of protein

Suspension cells ExpiCHO^{™} in logarithmic growth phase were prepared, and cultured in a cell shaker at 125 rpm, 37°C, 8% CO₂ until the cells had a density of 6×10⁶ cells/mL and viability of >98%. 25 mL of the cells were taken and placed in a new cell culture flask as a transfection system. Tube A: 1 mL of ExpiCHOTM Expresssion Medium containing 25 µg of plasmid; Tube B: 1 mL of ExpiCHOTM Expresssion Medium containing 80 µL of the transfection reagent in ExpiFectamine TM CHO Transfection Kit (Manufacturer: Thermo Scientific; Cat. No.: A29129). The media of Tube A and tube B were mixed, allowed to stand at room temperature for 2 min, and then the mixture was poured into 25 mL of the prepared transfection cell system. The cells were cultured in a cell shaker at 125 rpm, 37°C, 8% CO₂ for 18 to 22 h. 150 µL of the enhancer and 4 mL of the excipient from the ExpiFectamine^{™} CHO Transfection Kit were added to each flask, and cultured in a cell shaker at 125 rpm, 32°C, and 5% CO₂ for 8 to 15 days. After the culture was completed, centrifugation was performed at 4°C, 4000 rpm for 10 minutes, and the cell supernatant was collected.

The supernatant was filtrated with a 0.22 µm filter. AKTA instrument was turned on, the channels A and B were first rinsed with solution A (200 mM sodium hydrogen phosphate dodecahydrate) and solution B (100 mM citric acid monohydrate), and the protein A column was installed. The protein A column was equilibrated with solution A at a flow rate of 8 mL/min for more than 15 minutes, and the next step was proceeded after the UV value, pH value, and conductivity detected by the instrument were stable. The sample was loaded at a flow rate of 6 to 10 ml/min, and then the UV value would rise. This peak was the breakthrough peak, and the column was washed continuously with solution A, while the breakthrough peak sample was collected for detection. After the pH value was no longer changed, solution B was injected at a flow rate of 6 to 10 ml/min. Then the pH value decreased, the UV value increased, and this peak was the elution peak, and the antibody was mainly present in the elution peak. The elution peak sample was collected for detection. The column was equilibrated with solution A, then the pipe and protein A column were filled with 20% ethanol, and then the column was taken down, and stored at 4°C. The samples of the breakthrough peak and the elution peak were purified, and subjected to SDS-PAGE identification (the samples were boiled in water for 5 min, which could open the disulfide bonds between the heavy chain and light chain of the antibody, see Molecular Cloning: A Laboratory Manual, 3^{rd} Edition). The purified monoclonal antibody was dialyzed overnight with 20 mM PBS buffer, measured by ultraviolet spectrometry or BCA to determine its concentration, then dispensed into 1.5 ml tubes, and stored at -20°C for later use.

### Example 13. Polyacrylamide gel electrophoresis

On the first night, 293T cells were inoculated into a 6-well plate at 8×10⁵/well. On the second day, plasmid transfection was performed when the cell confluence reached 85%. 125µL of opti-MEM (manufacturer: Gibco; Cat. No.: 31985 to 070) medium was taken and added into a 1.5mL centrifuge tube, added with 2µg of plasmid, 4µL of P3000, vortexed and mixed, and marked as A. Another 125µL of opti-MEM medium was taken and added into another 1.5mL centrifuge tube, added with 4µL of Lipofectamine 3000 (manufacturer: Invitrogen; Cat. No.: L3000015), vortexed and mixed, and marked as B. The mixture in tube A was added to tube B, vortexed and mixed, and allowed to stand for 15 minutes. Then the solution was added to the 6-well plate with 293T cells, shaken gently, and placed in a cell culture incubator for culture. On the third day, the culture medium was discarded, and the cells were washed twice by adding pre-cooled PBS. 150µL of RIPA lysis buffer containing protease inhibitor as added, and the cells were lysed on ice for 30 minutes. The supernatant was collected after centrifugation at 12000rpm and 4°C for 20 minutes. The protein concentration of the supernatant was determined using BCA quantitative reagent (manufacturer: PIERCE; Cat. No.: 23225). The sample loading amount for electrophoresis was 20µg/well, β-mercaptoethanol and loading buffer were added to the sample, and heat denaturation was performed at 100°C for 10 minutes. A commercially available 12% precast gel was added, and the electrophoresis was performed at a voltage of 180V for 40 minutes. After electrophoresis, the SDS-PAGE gel was stained with 0.25% Coomassie Brilliant Blue (Sigma) overnight. After that, decolorization was performed with decolorization buffer (300 ml of methanol, 100 ml of acetic acid and 600 ml of double distilled water).

Result analysis: The molecular weight of the control protein SC-TM was around 60kDa, and the molecular weights of the mutated F protein truncates were smaller, about 30kDa. Fig. 9 showed the polyacrylamide gel electrophoresis results of SC-TM and LC2A.

### Example 14. Analysis of epitope characteristics of mutated F protein truncates

SC-TM and the 7 mutants were diluted with PBS buffer to prepare coating solutions with a final concentration of 1µg/mL. 100µL of the diluted coating solution was added to a 96-well ELISA plate, and coated overnight at 4°C. The plate was washed once with PBST washing solution (20 mM PB7.4, 150 mM NaCl, 0.1% Tween20), and spin-dried. Then 200µL of blocking solution (containing 20% calf serum and 1% casein, 20 mM Na₂HPO₄/NaH₂PO₄ buffer solution with a pH of 7.4) was added to each well, blocking was performed at 37°C for 2 hours, and then the blocking solution was discarded. After drying, the plate was packed into an aluminum foil bag, and stored at 4°C for later use. The F protein-specific monoclonal antibodies MOTA, D25, and hRSV90 (the VH and VL sequences of the three antibodies were set forth in SEQ ID NO: 36 to SEQ ID NO: 41 in Table 1, respectively, in which the three antibodies had the same heavy chain constant region with the specific sequence as set forth in SEQ ID NO: 42, and the three antibodies had the same light chain constant region with the specific sequence as set forth in SEQ ID NO: 43) that recognized different epitopes were taken, and subjected to 5 times gradient dilution with PBS solution starting from 100 µg/ml as the initial concentration for a total of 8 gradients, respectively. The coated ELISA plate was taken, added with the diluted antibody sample at 100 µl per well, and placed in a 37 °C incubator for reaction for 60 minutes. The ELISA plate was washed 5 times with PBST washing solution (20 mM PB7.4, 150 mM NaCl, 0.1% Tween20), added with horseradish peroxidase (HRP)-labeled goat anti-human IgG reaction solution at 100 µl per well, and placed in a 37 °C incubator for reaction for 30 minutes. After the enzyme label reaction step was completed, the ELISA plate was washed 5 times with PBST washing solution (20mM PB7.4, 150 mM NaCl, 0.1% Tween20), added with TMB colorimetric agent (purchased from Beijing Wantai Biological Pharmaceutical Co., Ltd.) at 50 µl per well, and placed in a 37°C incubator for reaction for 15 minutes. After the color development reaction step was completed, 50 µl of stop solution (purchased from Beijing Wantai Biological Pharmaceutical Co., Ltd.) was added to each well of the ELISA plate, and the OD450/630 values of each well were detected by a microplate reader.

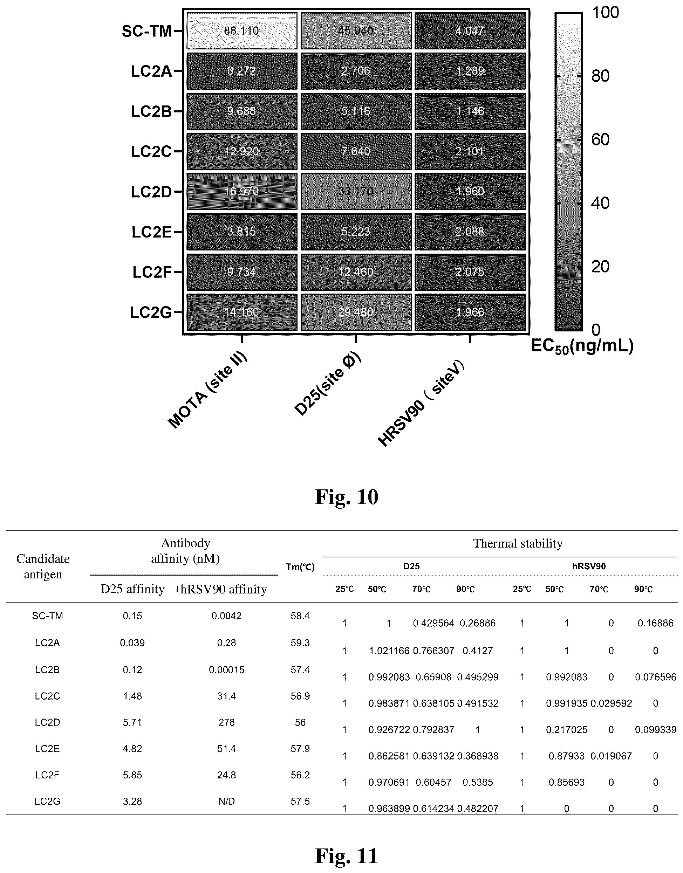

Result analysis: The results showed that the binding ability of several mutated F protein truncates with the test antibodies was better than that of SC-TM, in which especially the binding ability of LC2A, LC2B and LC2E with high-neutralization antibodies D25 and hRSV90 was significantly enhanced (Fig. 10).

### Example 15. Analysis of affinity of mutated F protein truncates with antibodies

The binding affinity of the mutated F protein truncates with F protein-specific antibodies D25 and hRSV90 was determined by surface plasmon resonance analysis (SPR) technology. The mutated F protein truncates with histidine tags were immobilized at a concentration of about 100 nM on NTA sensor chips (Cytiva) using Biacore-8K (Cytiva). The gradiently diluted antibodies (with a concentration ranged from 200 to 0.78 nM) were added. RU data were fitted to a 1:1 binding model using Biacore^{™} Insight software.

Result analysis: The results showed that the affinity of several mutated F protein truncates to the test antibody was comparable to that of SC-TM, in which the affinity of LC2A to D25 was 3.85 times higher than that of SC-TM, the affinity of LC2B to D25 was 1.25 times higher than that of SC-TM, and the affinity of LC2B to hRSV90 was 3.85 times higher than that of SC-TM (Fig. 11).

### Example 16. Analysis of thermal stability of mutated F protein truncates

(1) Differential Scanning Fluorimetry (DSF) was used to analyze the thermal stability of SC-TM and the 7 mutated F protein truncates. First, the protein sample to be tested was diluted to a concentration of 1 mg/mL with sterile PBS solution, then subjected to 2 times gradient dilution for a total of 8 gradients, so as to determine the appropriate sample concentration. Blank PBS was used as a control. The dye SYPRO Orange (manufacturer: SIGMA-ALDRICH; Cat. No.: S5692-500UL) was diluted with deionized water to a final concentration of 50X. 45µL of the diluted protein sample to be tested was taken and added to a Bio-Rad PCR plate, then 5µL of 50X SYPRO Orange dye was added. The above-configured system was placed in a fluorescence quantitative PCR instrument (BIO-RAD), the excitation light and emission light wavelengths were adjusted to the corresponding wavelengths of the fluorescent dye, the heating rate was set to 0.5°C/10s, and the temperature range was from 0°C to 100°C. BIO-RAD manager was used to process the data, the curve of fluorescence intensity versus temperature was plotted, and the Tm value of the protein sample was calculated by fitting Boltzmann transport equation (BTE).
   Result analysis: The results showed that as compared to the control protein pre-F, there was no significant difference in the thermal stability of the 7 mutated F protein truncates, among which the Tm value of LC2A was slightly higher than that of SC-TM (59.3°C vs. 58.4°C) (Fig. 11).
(2) The epitope thermal stability of SC-TM and 7 mutated F protein truncates was analyzed by enzyme-linked immunosorbent assay (ELISA). The protein to be tested was diluted to 10 µg/mL using PBS solution, and placed at 25°C, 50°C, 70°C and 90°C for 1 hour, respectively, and then immediately cooled on ice. The coating and detection steps were consistent with those described in Example 4.

Result analysis: The results showed that the binding of the control protein SC-TM and the 7 mutated F protein truncates to D25 could still be detected at 70°C and 90°C, and the binding of the 7 mutants was better than that of SC-TM (Fig. 11).

### Example 17. Analysis of immunogenicity of mutated F protein truncates

### (1) Experiment of immunization in mice

The feeding and experimental operations of experimental animals were carried out in accordance with the procedures established by the Experimental Animal Use and Management Committee of Xiamen University. The experimental animals were BALB/c female mice purchased from Beijing Weitong Lihua Experimental Animal Technology Co., Ltd., 6 to 8 weeks old, and were raised at the Experimental Animal Center of Xiamen University. The immunization scheme was that SC-TM and the 7 mutated F protein truncates were separately mixed with aluminum adjuvant in a volume ratio of 1: 1 for immunization of mice. Each group consisted of 5 mice, the immunization method was unilateral thigh muscle injection, and the immunization dose was 20 µg protein/mouse. The immunization were performed by two injections, and orbital blood was collected from the mice on the 7th day after the booster immunization. The serum was separated, and the serum sample was inactivated at 56°C for 30 minutes and placed at 4°C for later use.

### (2) Evaluation of specific antibody titers of pre-F and post-F proteins in serum

The pre-F protein used for coating was SC-TM, and the sequence of post-F protein was downloaded from GenBank (GenBank ID: 6APB_A, SEQ ID NO: 44). The nucleotide sequence encoding post-F protein was cloned into the pcDNA3.1 eukaryotic expression vector and expressed using the ExpiCHO^{™} system. The expression steps were the same as those described in Example 3. The protein coating and blocking steps were the same as those described in Example 4. The serum was subjected to 5 times gradient dilution, starting from 200 times in the first well, for a total of 8 gradients. The diluted serum was added to the blocked 96-well plate at a volume of 100 µL/well and incubated at 37°C for 1 hour. The serum was discarded, the plate was washed 5 times with PBST, and spin-dried. Horseradish peroxidase-labeled goat anti-mouse IgG antibody (manufacturer: Abcam; Cat. No.: ab97265) diluted at 1:5000 was added at a volume of 100 µL/well and incubated at 37°C for 1 hour. The secondary antibody was discarded, and the plate was washed 5 times with PBST. The color development solution was added at a volume of 100 µL/well, and the color development was performed at room temperature in the dark for 10 minutes. The stop solution was added at a volume of 50 µL/well, and the absorbance at 450nm was detected by a microplate reader. The judgment standard for the endpoint titer was: 3 times the reading value of the negative well.

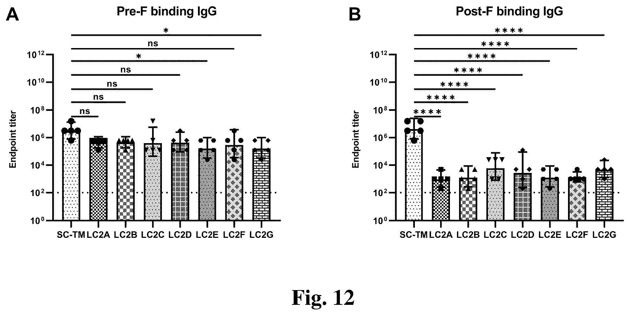

Result analysis: The results showed that all the 7 mutated F protein truncates could induce the antibody response with pre-F bias, in which the pre-F binding antibody titers induced by LC2A, LC2B, LC2C, LC2D and LC2F were comparable to that of the full-length pre-F protein SC-TM (Fig. 12).

Although the specific embodiments of the present invention have been described in detail, those skilled in the art will understand that various modifications and changes can be made to the details based on all the teachings disclosed, and these changes are within the scope of protection of the present invention. The entirety of the present invention is given by the appended claims and any equivalents thereof.

## Claims

1. A fusion protein, comprising a first truncate, a second truncate, and a linker for linking the first truncate and the second truncate; wherein,
as compared to the F2 protein of wild respiratory syncytial virus (RSV), the first truncate has 5 to 25 amino acids (e.g., 5 to 8, 9 to 12, 13 to 16, 17 to 20, 21 to 25) truncated at the N-terminal, and 3, 4 or 5 amino acids truncated at the C-terminal of the F2 protein of wild RSV;
as compared to the F1 protein of wild RSV, the second truncate has 7, 8 or 9 amino acids truncated at the N-terminal, and 238 to 268 amino acids (e.g., 238 to 241, 242 to 245, 246 to 249, 250 to 253, 254 to 257, 258 to 261, 262 to 265, 266 to 268) truncated at the C-terminal of the F1 protein of wild RSV.

2. The fusion protein according to claim 1, wherein compared to the F2 protein of wild RSV, the first truncate has 5 or 25 amino acids truncated at the N-terminal and 4 amino acids truncated at the C-terminal of the F2 protein of wild RSV;
preferably, the F2 protein of wild RSV has an amino acid sequence as set forth in SEQ ID NO: 13;
preferably, the first truncate has an amino acid sequence as set forth in SEQ ID NO: 16 or 17.

3. The fusion protein according to claim 1 or 2, wherein compared to the F1 protein of wild RSV, the second truncate has 8 or 9 amino acids truncated at the N-terminal and 252 or 268 amino acids truncated at the C-terminal of the F1 protein of wild RSV;
preferably, the F1 protein of wild RSV has an amino acid sequence as set forth in SEQ ID NO: 12;
preferably, the second truncate has an amino acid sequence as set forth in SEQ ID NO: 14 or 15.

4. The fusion protein according to any one of claims 1 to 3, wherein the linker comprises at least one (e.g., 1, 2) proline;
preferably, the linker has 3 to 20 (e.g., 3, 4, 5, 6, 7, 8, 9, 10, 15, 20) amino acids; more preferably, the linker has 5 to 8 amino acids;
preferably, the linker comprises a plurality of (e.g., 2, 3, 4, 5, 6, 7) glycine and at least one (e.g., 1, 2, 3) proline;
preferably, the linker has a sequence as set forth in SEQ ID NO: 2;
preferably, the first truncate is located at the N-terminal of the linker;
preferably, the second truncate is located at the C-terminal of the linker;
preferably, the fusion protein comprises, from the N-terminal to the C-terminal, the first truncate, the linker, and the second truncate;
preferably, the fusion protein has an amino acid sequence as set forth in SEQ ID NO: 3 or 4.

5. The fusion protein according to any one of claims 1 to 4, wherein the fusion protein further comprises a substitution, deletion or addition of one or more amino acids (e.g., a substitution, deletion or addition of 1, 2, 3, 4, 5, 6, 7, 8, 9 or 10 amino acids);
preferably, the first truncate and/or the second truncate of the fusion protein comprises a substitution, deletion or addition of one or more amino acids (e.g., a substitution, deletion or addition of 1, 2, 3, 4, 5, 6, 7, 8, 9 or 10 amino acids);
preferably, the sequence of the fusion protein has a substitution, deletion or addition of one or more amino acids (e.g., a substitution, deletion or addition of 1, 2, 3, 4, 5, 6, 7, 8, 9 or 10 amino acids) as compared to the sequence as set forth in any one of SEQ ID NOs: 3 to 7;
preferably, the substitution is a conservative substitution.

6. The fusion protein according to claim 5, wherein the sequence of the fusion protein has a substitution, deletion or addition of one or more amino acids (e.g., a substitution, deletion or addition of 1, 2, 3, 4, 5, 6, 7, 8, 9 or 10 amino acids) as compared to the sequence as set forth in SEQ ID NO: 4;
preferably, as compared to the sequence as set forth in SEQ ID NO: 4, the sequence of the fusion protein has a substitution of amino acid at the position corresponding to the position 29 of SEQ ID NO: 4, and the sequence of the fusion protein also has a substitution of amino acid at any one of the positions corresponding to the positions 47 to 214 of SEQ ID NO: 4;
preferably, as compared to the sequence as set forth in SEQ ID NO: 4, the sequence of the fusion protein has a substitution of amino acid at the position corresponding to the position 114 of SEQ ID NO: 4, and the sequence of the fusion protein also has a substitution of amino acid at any one of the positions corresponding to the positions 29 to 94 of SEQ ID NO: 4
preferably, as compared to the sequence as set forth in SEQ ID NO: 4, the sequence of the fusion protein has a substitution of one or more amino acids at the following positions corresponding to the position 29, position 47, position 212, position 214, position 88, position 94, position 114 and position 101 of SEQ ID NO: 4;
preferably, the fusion protein has one or more characteristics selected from the following:
(1) as compared to the sequence as set forth in SEQ ID NO: 4, the sequence of the fusion protein has a substitution of amino acid at the positions corresponding to the positions 29 and 212 of SEQ ID NO: 4;
(2) as compared to the sequence as set forth in SEQ ID NO: 4, the sequence of the fusion protein has a substitution of amino acid at the positions corresponding to the positions 29 and 214 of SEQ ID NO: 4;
(3) as compared to the sequence as set forth in SEQ ID NO: 4, the sequence of the fusion protein has a substitution of amino acid at the positions corresponding to the position 29 and position 114 of SEQ ID NO: 4;
(4) as compared to the sequence as set forth in SEQ ID NO: 4, the sequence of the fusion protein has a substitution of amino acid at the positions corresponding to the position 29, position 47, position 101 and position 214 of SEQ ID NO: 4;
(5) as compared to the sequence as set forth in SEQ ID NO: 4, the sequence of the fusion protein has a substitution of amino acid at the positions corresponding to the position 88, position 101, position 114 and position 214 of SEQ ID NO: 4;
(6) as compared to the sequence as set forth in SEQ ID NO: 4, the sequence of the fusion protein has a substitution of amino acid at the positions corresponding to the positions 94 and position 114 of SEQ ID NO: 4.

7. The fusion protein according to claim 6, wherein the fusion protein has one or more characteristics selected from the following:
(1) the substitution at position 29 is to substitute isoleucine I with leucine L;
(2) the substitution at position 212 is to substitute valine V with methionine M;
(3) the substitution at position 214 is to substitute alanine A with leucine L;
(4) the substitution at position 94 is to substitute valine V with isoleucine I;
(5) the substitution at position 47 is to substitute methionine M with alanine A;
(6) the substitution at position 114 is to substitute tyrosine Y with isoleucine I;
(7) the substitution at position 101 is to substitute valine V with isoleucine I;
(8) the substitution at position 214 is to substitute alanine A with leucine L;
(9) the substitution at position 88 is to substitute amino acid leucine L with alanine A;
preferably, the fusion protein has an amino acid sequence as set forth in any one of SEQ ID NOs: 28 to 34.

8. The fusion protein according to any one of claims 1 to 7, wherein the fusion protein further comprises: a signal peptide, a multimerization motif (e.g., a trimerization motif) and/or a membrane anchoring region;
preferably, the membrane anchoring region has an amino acid sequence as set forth in SEQ ID NO: 8;
preferably, the signal peptide has an amino acid sequence as set forth in SEQ ID NO: 9;
preferably, the multimerization motif has an amino acid sequence as set forth in SEQ ID NO: 21 or 22;
preferably, the signal peptide is located at the N-terminal of the first truncate; preferably, the signal peptide is linked to the first truncate via a first linker peptide;
preferably, the membrane anchoring region is located at the C-terminal of the second truncate; preferably, the membrane anchoring region is linked to the second truncate via a second linker peptide;
preferably, the multimerization motif is located at the C-terminal of the second truncate; preferably, the multimerization motif is linked to the second truncate via a second linker peptide;
preferably, the first linker peptide and the second linker peptide each independently contain one or several (e.g., 1, 2 or 3) sequences represented by (GₘS)ₙ, wherein m is an integer selected from 1 to 6, and n is an integer selected from 1 to 6; preferably, m is 3, 4, or 5; preferably, n is 1 or 2; preferably, the first linker peptide and the second linker peptide have the sequence as set forth in SEQ ID NO: 18;
preferably, the fusion protein comprises, from the N-terminal to the C-terminal, the signal peptide, the first linker peptide, the first truncate, the linker, the second truncate, the second linker peptide, and the membrane anchoring region;
preferably, the fusion protein has a sequence as set forth in SEQ ID NO: 19, 20, 45 or 46.

9. A nucleic acid molecule, comprising a nucleotide sequence encoding the fusion protein according to any one of claims 1 to 8;
preferably, the nucleotide sequence is codon-optimized or not codon-optimized according to the codon preference of a host cell.

10. A vector, comprising the nucleic acid molecule according to claim 9;
preferably, the vector is a viral vector;
preferably, the viral vector is selected from the group consisting of: influenza virus vector, enterovirus vector, retrovirus vector, adenovirus vector, adeno-associated virus vector, herpes virus vector, poxvirus vector, baculovirus vector, papillomavirus vector, or papovavirus vector.

11. A host cell, comprising the fusion protein according to any one of claims 1 to 8 or the nucleic acid molecule according to claim 9 or the vector according to claim 10;
preferably, the host cell is selected from the group consisting of prokaryotic cell (e.g., *Escherichia coli* cell) and eukaryotic cell;
preferably, the eukaryotic cell is a mammalian cell, such as a mouse cell, a human cell;
preferably, the fusion protein is displayed on the surface of the cell membrane of the host cell.

12. A method for expressing or producing the fusion protein according to any one of claims 1 to 8, the method comprising culturing the host cell according to claim 11 under a condition that allows protein expression, and optionally, recovering or purifying the expressed fusion protein.

13. A kit, comprising an antigen component and a carrier component capable of displaying the antigen component; wherein,
the antigen component comprises (i) the fusion protein according to any one of claims 1 to 8, (ii) the nucleic acid molecule according to claim 9, and/or (iii) an mRNA product transcribed from the nucleic acid molecule according to claim 9;
the carrier component is selected from the group consisting of: nanomaterial (e.g., lipid nanoparticle, protein nanoparticle, polymer nanoparticle, inorganic nanocarrier and biomimetic nanoparticle), bacterial outer membrane vesicles (OMVs), multimerization motif, virus-like particle (VLP), or any combination thereof;
preferably, the antigen component and the carrier component in the kit are provided separately or in a complex formed therefrom;
preferably, the antigen component is a monomer or a multimer (e.g., a dimer, a trimer, a tetramer, a pentamer);
preferably, the multimerization motif and/or the VLP in the kit are provided in the form of a protein or a nucleic acid;
preferably, the multimerization motif has an amino acid sequence as set forth in SEQ ID NO: 21 or 22;
preferably, the VLP is assembled by proteins obtained from RSV, hepatitis E virus (HEV), hepatitis B virus (HBV), human papillomavirus (HPV), or human immunodeficiency virus (HIV).

14. A pharmaceutical composition, comprising a pharmaceutically acceptable carrier and/or excipient, and any one or more selected from the following items (1) to (4):
(1) the fusion protein according to any one of claims 1 to 8;
(2) the nucleic acid molecule according to claim 9;
(3) the vector according to claim 10;
(4) the host cell according to claim 11.

15. A vaccine, comprising the fusion protein according to any one of claims 1 to 8, or the nucleic acid molecule according to claim 9, or an mRNA product transcribed from the nucleic acid molecule according to claim 9, or the vector according to claim 10;
preferably, the vaccine being prepared by the kit according to claim 13;
preferably, the vaccine further comprising an adjuvant.

16. A method for inducing an antibody against RSV, the method comprising administering (e.g., injecting) into a cell in vitro or a subject an effective amount of the fusion protein according to any one of claims 1 to 8, or the nucleic acid molecule according to claim 9, or the vector according to claim 10, or the host cell according to claim 11, or the pharmaceutical composition according to claim 14, or the vaccine according to claim 15;
preferably, the antibody is a neutralization antibody.

17. A method for detecting the presence of RSV infection in a subject in vitro, the method comprising: contacting a biological sample obtained from the subject with the fusion protein according to any one of claims 1 to 8; and detecting the presence of a complex formed by the fusion protein and an antibody from the biological sample;
preferably, the subject is a mammal, such as a mouse or a human;
preferably, the biological sample is selected from the group consisting of whole blood, serum, plasma, or any combination thereof.

18. A method for screening a candidate medicament capable of inhibiting RSV infection in a cell, the method comprising contacting a host cell with the candidate medicament before, simultaneously or after contacting the fusion protein according to any one of claims 1 to 8, or the vector according to claim 10, or the pharmaceutical composition according to claim 14, or the vaccine according to claim 15 with the host cell.

19. Use of the fusion protein according to any one of claims 1 to 8, or the nucleic acid molecule according to claim 9, or the vector according to claim 10, or the host cell according to claim 11, or the pharmaceutical composition according to claim 14, or the vaccine according to claim 15, in the manufacture of a kit for inducing an immune response to RSV in a subject;
preferably, the immune response comprises inducing the subject to produce an antibody against RSV;
preferably, the antibody is a neutralization antibody;
preferably, the subject is a mammal, such as a mouse or a human.

20. Use of the fusion protein according to any one of claims 1 to 8, or the nucleic acid molecule according to claim 9, or the vector according to claim 10, or the host cell according to claim 11, or the pharmaceutical composition according to claim 14, or the vaccine according to claim 15, in the manufacture of a kit for preventing and/or treating a RSV infection or a disease and/or symptom caused by RSV infection;
preferably, the subject is a mammal, such as a mouse or a human;
preferably, the disease and symptom caused by RSV infection is selected from the group consisting of bronchiolitis, pneumonia, asthma, obstructive pulmonary disease, cardiopulmonary complication.
